# EUROPÄISCHE PATENTSCHRIFT

(11) **EP 1 546 319 B1**
(45) Veröffentlichungstag und Bekanntmachung des Hinweises auf die Patenterteilung: **28.11.2007**
(21) Anmeldenummer: 03807840.8
(22) Anmeldetag: 02.10.2003
(51) Int. Cl.: C12N 9/88, C12N 15/60, C12P 3/00, C12N 15/11, G01N 33/50, C12N 15/62

(54) **ABBAU UND MODIFIZIERUNG VON SILICATEN UND SILICONEN DURCH SILICASE UND VERWENDUNG DES REVERSIBLEN ENZYMS**
DECOMPOSITION AND MODIFICATION OF SILICATE AND SILICONE BY SILICASE AND USE OF THE REVERSIBLE ENZYME
DECOMPOSITION ET MODIFICATION DE SILICATES ET DE SILICONES PAR LA SILICASE ET UTILISATION DE L'ENZYME REVERSIBLE

(30) Priorität: 03.10.2002 DE 10246186
(43) Veröffentlichungstag der Anmeldung: 29.06.2005
(73) Patentinhaber: Johannes Gutenberg-Universität Mainz, 55122 Mainz (DE)
(72) Erfinder: MÜLLER, Werner, E., G., 65203 Wiesbaden (DE); SCHRÖDER, Heinz, 65189 Wiesbaden (DE); KRASKO, Anatoli, 55116 Mainz (DE)
(74) Vertreter: Krauss, Jan
(86) Internationale Anmeldenummer: PCT/EP2003/010983
(87) Internationale Veröffentlichungsnummer: WO 2004/033679

(56) Entgegenhaltungen:
- WO-A-00/35993
- SCHRÖDER HEINZ C ET AL: "Silicase, an enzyme which degrades biogenous amorphous silica: contribution to the metabolism of silica deposition in the demosponge Suberites domuncula." PROGRESS IN MOLECULAR AND SUBCELLULAR BIOLOGY. UNITED STATES 2003, Bd. 33, 2003, Seiten 249-268, XP008027318 ISSN: 0079-6484
- DATABASE EMBL [Online] 26. Januar 1992 (1992-01-26) VENTA PJ ET AL.: "H.sapiens carbonic anhydrase II (CAII) gene, exon 7, and complete cds." retrieved from EBI Database accession no. M77181 XP002269513
- BUDAVARI S (ED) : "The Merck Index, twelfth edition " 1996 , MERCK , WHITEHOUSE STATION XP002269512 ISBN: 0911910-12-3 Entry 8634 Seite 1459
- KRASKO: "Expression of silicatein and collagen genes in the marine sponge Suberites domuncula is controlled by silicate and myotrophin" EUROPEAN JOURNAL OF BIOCHEMISTRY, BERLIN, DE, Bd. 267, August 2000 (2000-08), Seiten 4878-4887, XP002190292 ISSN: 0014-2956 in der Anmeldung erwähnt
- SHIMIZU K ET AL: "SILICATEIN ALPHA: L-LIKE PROTEIN IN SPONGE BIOSILICA" PROCEEDINGS OF THE NATIONAL ACADEMY OF SCIENCES OF USA, NATIONAL ACADEMY OF SCIENCE. WASHINGTON, US, Bd. 95, Mai 1998 (1998-05), Seiten 6234-6238, XP002924682 ISSN: 0027-8424
- CHA J N ET AL: "SILICATEIN FILAMENTS AND SUBUNITS FROM A MARINE SPONGE DIRECT THE POLYMERIZATION OF SILICA AND SILICONES IN VITRO" PROCEEDINGS OF THE NATIONAL ACADEMY OF SCIENCES OF USA, NATIONAL ACADEMY OF SCIENCE. WASHINGTON, US, Bd. 96, Januar 1999 (1999-01), Seiten 361-365, XP001037314 ISSN: 0027-8424 in der Anmeldung erwähnt
- BRAWER A E: "Silicon and matrix macromolecules: New research opportunities of old diseases from analysis of potential mechanisms of breast implant toxicity" MEDICAL HYPOTHESES, Bd. 51, Nr. 1, Juli 1998 (1998-07), Seiten 27-35, XP008027327 ISSN: 0306-9877

### SEQUENCE LISTING

<110> Johannes Gutenberg Universität Mainz
   <120> Abbau von Silikaten und Siliconen durch Silicase und Verwendung des reversiblen Enzyms
<130> U30033PCT
   <160> 2
   <170> Patent In version 3.1
<210> 1
   <211> 1396
   <212> DNA
   <213> Suberites domuncula
   <400> 1
   gaattcggca cgagggacaa ctttgcataa cttttactgt ccatgtttaa cgtttagatc      60
   tagtactagt agtctacaag aacaactgtc aacaactgtc agattatgtg tataaaccaa     120
   gatgtctgca attcttaaga gaaacgtacc tatccaaaga gtcggtctcc cactgacctc     180
   ctatgtcagt agatgggctt ctgctctgcc caccaggacc catccttttt acaagttggt     240
   tgatgacagt accaccccag tgacaaggtc tactcttctc agtgctcata tggttgacac     300
   cttgctagat gagaaccagc agagcagaca tgaaaaccaa cacacagaca cgtcttacaa     360
   aatgtaccag ggattaaaat ttgttgtaaa gacgctgttt actccatcga aatgccaccg     420
   tcacttctcc acatcagctc atttgtctgc catgggtcga catcaatccc ccatcaatat     480
   aatcacctcc agtacgacca aaggaccgtc attgaaaccg ttaaaattta gcaagagttg     540
   ggacaagcca gtaatcggca ccgtcaaaga tactggctat tatcttaaat ttgcaccaga     600
   atctgcagca gagaagtgca cattgcatac gtacaatggt gaatatatcc tagatcattt     660
   ccattatcac tgggggaaga aggatgggga aggagcagag catttcatcg atggaaaaca     720
   atacgacatc gagttccact ttgtacataa aaaggttggg ttgactgatc cagatgctag     780
   agacgctttt gctgttttgg gcgtttttgg aaaggccgac cctcgtttga agatcaatgg     840
   aatctgggag ctactctcac cgtcaactgt cctgactgtc gactcaacac gaaacgtcgc     900
   tgatgttgtt ccctctaagc ttctcccaag tgccagagac tattttcact atgaaggttc     960
   tttgaccaca cctacgtatg gtgaggttgt gcactggttt gttctcaatg aacccatagc    1020
   tgtccctagt gagtatctgt cagctctgag acagatgcaa gctgacaaag aaggtactgt    1080
   gattgactca aactatcgag agcttcaaga agtccacaat cgacctgtgc aacgatttaa    1140
   gagtgatgag caagggagag gagaatttga cgatatttct aagaatgagg acattgtgga    1200
   ggacttgtct aaattgtctg gtaactttat tagagagctg gtcaggaaga tatattggtg    1260
   acctttttct acacttgtta gagttttagg ccagaataca tttcatcatt tggactgtta     1320
   ttttgtgtac actgcttagc agtttatata aacactacaa tgccattatt ataatatagc    1380
   caatgctgtg atttga                                                    1396
<210> 2
   <211> 379
   <212> PRT
   <213> Suberites domuncula
<400> 2
   Met Ser Ala Ile Leu Lys Arg Asn Val Pro Ile Gln Arg Val Gly Leu
   1               5                   10                  15
   Pro Leu Thr Ser Tyr Val Ser Arg Trp Ala Ser Ala Leu Pro Thr Arg
               20                  25                  30
   Thr His Pro Phe Tyr Lys Leu Val Asp Asp Ser Thr Thr Pro Val Thr
           35                  40                  45
   Arg Ser Thr Leu Leu Ser Ala His Met Val Asp Thr Leu Leu Asp Glu
       50                  55                  60
   Asn Gln Gln Ser Arg His Glu Asn Gln His Thr Asp Thr Ser Tyr Lys
   65                  70                  75                  80
   Met Tyr Gln Gly Leu Lys Phe Val Val Lys Thr Leu Phe Thr Pro Ser
                   85                  90                  95
   Lys Cys His Arg His Phe Ser Thr Ser Ala His Leu Ser Ala Met Gly
               100                 105                 110
   Arg His Gln Ser Pro Ile Asn Ile Ile Thr Ser Ser Thr Thr Lys Gly
           115                 120                 125
   Pro Ser Leu Lys Pro Leu Lys Phe Ser Lys Ser Trp Asp Lys Pro Val
       130                 135                 140
   Ile Gly Thr Val Lys Asp Thr Gly Tyr Tyr Leu Lys Phe Ala Pro Glu
   145                 150                 155                 160
   Ser Ala Ala Glu Lys Cys Thr Leu His Thr Tyr Asn Gly Glu Tyr Ile
                   165                 170                 175
   Leu Asp His Phe His Tyr His Trp Gly Lys Lys Asp Gly Glu Gly Ala
               180                 185                 190
   Glu His Phe Ile Asp Gly Lys Gln Tyr Asp Ile Glu Phe His Phe Val
           195                 200                 205
   His Lys Lys Val Gly Leu Thr Asp Pro Asp Ala Arg Asp Ala Phe Ala
       210                 215                 220
   Val Leu Gly Val Phe Gly Lys Ala Asp Pro Arg Leu Lys Ile Asn Gly
   225                 230                 235                 240
   Ile Trp Glu Leu Leu Ser Pro Ser Thr Val Leu Thr Val Asp Ser Thr
                   245                 250                 255
   Arg Asn Val Ala Asp Val Val Pro Ser Lys Leu Leu Pro Ser Ala Arg
               260                 265                 270
   Asp Tyr Phe His Tyr Glu Gly Ser Leu Thr Thr Pro Thr Tyr Gly Glu
           275                 280                 285
   Val Val His Trp Phe Val Leu Asn Glu Pro Ile Ala Val Pro Ser Glu
       290                 295                 300
   Tyr Leu Ser Ala Leu Arg Gln Met Gln Ala Asp Lys Glu Gly Thr Val
   305                 310                 315                 320
   Ile Asp Ser Asn Tyr Arg Glu Leu Gln Glu Val His Asn Arg Pro Val
                   325                 330                 335
   Gln Arg Phe Lys Ser Asp Glu Gln Gly Arg Gly Glu Phe Asp Asp Ile
               340                 345                 350
   Ser Lys Asn Glu Asp Ile Val Glu Asp Leu Ser Lys Leu Ser Gly Asn
           355                 360                 365
   Phe Ile Arg Glu Leu Val Arg Lys Ile Tyr Trp
       370                 375

## Beschreibung

### 1. Stand der Technik

Silicium ist das zweithäufigste Element der Erdkruste und kommt in verschiedenartigsten Verbindungen vor. Siliciumverbindungen stellen nicht nur die artenreichste Klasse der Mineralien dar, sondern sind auch in wirtschaftlicher Hinsicht bedeutsam. Technisch angewandte, aus Silicaten bestehende Materialien sind beispielsweise Glas, Porzellan, Email, Tonwaren, Zement und Wasserglas. Einige Silicate besitzen katalytische Eigenschaften. Durch teilweisen Austausch der von Silicium eingenommenen Gitterstellen gegen andere Elemente, insbesondere Aluminium, wird die Vielfalt in den möglichen Strukturen und technischen Einsatzmöglichkeiten noch erweitert. So besitzen die Alumosilicate, zu denen die Feldspäte und die Zeolithe gehören, Bedeutung u. a. auf Grund ihrer Molekularsieb- und lonenaustauscher-Eigenschaften. Weitere Silicium-Verbindungen, wie die Silicone (Siloxane), besitzen u. a. auch medizinische Bedeutung, wie zur Herstellung von Implantaten.

### 1.1. Siliciumdioxid

Siliciumdioxid (SiO₂) kommt sowohl in kristalliner als auch in amorpher Form vor. Zu den verschiedenen Formen des kristallinen SiO₂ gehören u. a. Quarz, Tridymit und Cristobalit. Achat, Opal und Feuerstein stellen amorphe Siliciumdioxid-Mineralien dar. In allen diesen Erscheinungsformen besitzt Silicium die Koordinationszahl 4 und ist tetraedrisch von vier Sauerstoff-Atomen umgeben.

Aus amorphem SiO₂ bestehen auch die Schalen von Kieselalgen (Diatomeen) und die Nadeln (Spiculae) von Kieselschwämmen.

### 1.2. Kieselsäuren und Silicate

Das tetraedrisch gebaute [SiO₄]⁴⁻-lon neigt zur Polymerisation durch Verknüpfung von SiO₄-Einheiten, wobei jeweils zwei Si-Atome über ein O-Atom miteinander verbunden werden. Durch Kondensation (Wasserabspaltung) entsteht dabei aus der Orthokieselsäure zunächst die Orthodikieselsäure (Pyrokieselsäure; H₆Si₂O₇). Die weitere Kondensation führt über die Polykieselsäuren zu den Metakieselsäuren [(H₂SiO₃)ₙ]. Bei kleinerer Zahl der SiO₄-Einheiten (n = 3, 4 oder 6) können sich dabei auch ringförmige Moleküle bilden.

Die wasserlöslichen Salze der Kieselsäuren, die Alkalisilicate, welche beispielsweise durch Schmelzen von Quarz mit Soda, Lauge oder Kaliumcarbonat gewonnen werden, enthalten neben [SiO₄]⁴⁻- auch [Si₂O₇]⁶⁻-, [Si₃O₁₀]⁸⁻- und größere Anionen. Nach Ansäuerung einer solchen Alkalisilicatlösung kondensieren die durch Protonenaufnahme gebildeten Säuremoleküle untereinander zu Polykieselsäuren, wobei die Lösung gelartig wird. Bei weiterem Fortschreiten der Kondensation entstehen aus den zunächst erhaltenen Ketten oder Netzen dreidimensionale Strukturen, welche der Zusammensetzung SiO₂ entsprechen.

Die Silicate lassen sich einteilen in .1.) Silicate mit diskreten Anionen, nämlich 1a.) Insel-Silicate (Ortho-Silicate mit dem Anion [SiO₄]²⁻; Beispiel: Phenakit, Olivin, Zirkon), 1b.) Gruppen-Silicate (Verknüpfung der SiO₄-Tetraeder zu kurzkettigen Einheiten; Beispiel: Di-Silicate und Tri-Silicate) und 1c.) Ring-Silicate (ringförmige Verknüpfung der SiO₄-Tetraeder; Beispiel: Benitoid mit 3-er-Ring, Axinit mit 4-er-Ring und Beryll mit 6-er-Ring), 2.) Ketten-Silicate und Band-Silicate (kettenartig miteinander verbundenen SiO₄-Tetraedern, die Polymere des Anions [SiO₃]²⁻ darstellen, und bandförmige Moleküle, die durch Verknüpfung mehrerer SiO₄-Ketten entstehen; Beispiele: Hornblenden, Asbeste), 3.) Schicht-oder Blatt-Silicate (aus ebenen Schichten von SiO₄-Tetraeder, die Polymere des Anions [Si₄O₁₀]⁴⁻ darstellen und durch dazwischen gelagerte Kationen zusammengehalten werden; Beispiel: Talk, Kaolinit) und 4.) Gerüstsilicate (Verknüpfung der tetraedrischen SiO₄-Gruppen zu dreidimensionalen Gittern; Beispiel: verschiedene Modifikationen von Siliciumdioxid wie Feldspäte).

Allgemeine Literatur: Hinz, Silicat-Lexikon (2 Bd.), Berlin: Akademie Verl. 1985; Liebau, Structural Chemistry of Silicates, Berlin: Springer 1985; Petzold und Hinz, Einführung in die Grundlagen der Silicatchemie, Stuttgart: Enke 1979; CD Römpp Chemie Lexikon - Version 1.0, Stuttgart/New York: Georg Thieme Verlag 1995.

### 1.3. Silicone

Durch teilweises Ersetzen der OH-Gruppen der Kieselsäure durch einbindige Organylreste, die sich nicht am Kondensationsvorgang beteiligen, entstehen verschiedene Silicone (Siloxane). Sie werden eingeteilt in: 1.) lineare Polysiloxane (Bautyp: R₃SiO[R₂SiO]ₙSiR₃), 2.) verzweigte Polysiloxane (mit trifunktionelle oder tetrafunktionelle Siloxan-Einheiten an den Verzweigungsstellen), 3.) cyclische Polysiloxane (aus difunktionellen Siloxan-Einheiten) und 4.) vernetzte Polymere (Verknüpfung von ketten- oder ringförmige Moleküle zu zwei- oder dreidimensionalen Netzwerken).

Silicone sind bedeutsame technische Werkstoffe. Die Viskosität der **kettenförmig aufgebauten Silicone (Siliconöle) nimmt mit wachsender Kettenlänge zu. Silicone, die in geringem Maß vernetzt sind,** zeigen Kautschuk-Elastizität (Siliconkautschuk), stark vernetzte Ketten sind harzartige (Siliconharze).

### 1.4. Biomineralisation (Bildung von biogenem Siliciumdioxid) in Keseischwämmen

Viele Siliciumverbindungen sind nur kostenintensiv herzustellen. Der Prozess der chemischen Synthese der Silicate bedarf oft drastischer Bedingungen wie hoher Druck und hohe Temperatur. Kieselschwämme sind dagegen - ebenso wie Kieselalgen - befähigt, mit Hilfe spezifischer Enzyme Silicatgerüste unter milden Bedingungen, d. h. bei relativ niedriger Temperatur und niedrigem Druck, zu bilden. Weiterhin ist die SiO₂-Synthese bei diesen Organismen durch hohe Spezifität, Regulierbarkeit und die Möglichkeit der Synthese von definierten Mikrostrukturen (Nanostrukturen) ausgezeichnet.

Hexactinellida (Glasschwämme) aus amorphem nichtkristallinem Siliciumdioxid bestehen. Die Demospongien und Hexactinellidae sind die einzigen Metazoen, die Siliciumdioxid anstelle von Calcium in ihrem Skelett besitzen.

Das opale Siliciumdioxid in den Spiculae der Kieselschwämme enthält 6-13% Wasser, was die ungefähre Formel (SiO₂)₂₋₅•H₂O ergibt (Schwab DW, Shore RE (1971) Mechanism of internal stratification of siliceous spicules. Nature 232:501-502).

Ein Enzym, das bei silicatbildenden Organismen an der Synthese des SiO₂-Skeletts beteiligt ist, und seine technische Verwendung wurden beschrieben (WO 00/35993. Methods, compositions, and biomimetic catalysts, such as silicateins and block copolypeptides, used to catalyze and spatially direct the polycondensation of silicon alkoxides, metal alkoxides, and their organic conjugates to make silica, polysiloxanes, polymetallo-oxanes, and mixed poly(silicon/ metallo)oxane materials under environmentally benign conditions. Inventors/ Applicants: Morse DE, Stucky GD, Deming, TD, Cha J, Shimizu K, Zhou Y; DE 100 37 270 A1. Silicatein-vermittelte Synthese von amorphen Silicaten und Siloxanen und ihre Verwendung. Deutsches Patentamt 2000. Anmelder und Erfinder: Müller WEG, Lorenz B, Krasko A, Schröder HC; WO 02/10420 A2. Silicatein-mediated synthesis of amorphous silicates and siloxanes and use thereof. Inventors/Applicants: Müller WEG, Lorenz B, Krasko A, Schröder HC). Dieses Enzym wurde aus dem marinen Kieselschwamm *Suberites domuncula* kloniert (Krasko A, Batel R, Schröder HC, Müller IM, Müller WEG (2000) Expression of silicatein and collagen genes in the marine sponge S. domuncula is controlled by silicate and myotrophin. Europ J Biochem 267:4878-4887). Das aus natürlichen Quellen isolierte Enzym ("Silicatein") ist in der Lage, aus organischen Siliciumverbindungen (Alkoxysilanen) amorphes Siliciumdioxid (Polykieselsäuren und Polysilicate) zu synthetisieren (Cha JN, Shimizu K, Zhou Y, Christianssen SC, Chmelka BF, Stucky GD, Morse DE (1999) Silicatein filaments and subunits from a marine sponge direct the polymerization of silica and silicones in vitro. Proc Natl Acad Sci USA 96:361-365).

### - EINFÜGUNG Seite 4a -

Unter der Datenbank-Zugangsnummer M77181 wird das Gen der *Homo sapiens* Carbonanhydrase II (abgekürzt als CAII) (Exon 7, und vollständige cds) geführt, dessen Sequenz von Venta *et al.* aufgeklärt wurde (siehe Venta,P.J., Welty, R.J., Johnson,T.M., Sly,W.S. and Tashian,R.E. (1991) Carbonic anhydrase II deficiency syndrome in a Belgian family is caused by a point mutation at an invariant histidine residue (107His----Tyr): complete structure of the normal human CA II gene. Am. J. Hum. Genet. 49(5):1082-1090.) Diese Gensequenz weist 40 % Übereinstimmung mit der Nukleinsäuresequenz der SEQ ID Nr, 1 und 34 % Übereinstimmung mit einem Teil (260 Aminosäuren) der SEQ ID Nr. 2 auf.

Krasko *et al.* (Krasko A, Lorenz B, Batel R, Schroder HC, Muller IM, Muller WE. (2000) Expression of silicatein and collagen genes in the marine sponge Suberites domuncula is controlled by silicate and myotrophin. Eur J Biochem. 267(15):4878-87.) beschreiben die Isolierung einer cDNA aus dem Schwamm *Suberites domuncula,* die für Silicatein kodiert, ein Enzym. das Alkoxid-Substrate zu Siliciumdioxid polymerisiert. Das Gen ist in starkem Maße hochreguliert, wenn die Konzentration von löslichem Silicat im Seewassermedium von Primmorphen von 1 µM auf ungefähr 60 µM erhöht wurde. Myotrophin hatte jedoch keinen Effekt auf die Expression des Silicateins.

Überraschenderweise konnte von den Erfindern - zunächst in dem Meeresschwamm *S. domuncula* als Modellsystem, ein Enzym (genannt: "Silicase") entdeckt werden, das in der Lage ist, sowohl amorphes als auch kristallines Siliciumdioxid aufzulösen.

Dieses Enzym, das am Katabolismus von Siliciumdioxid in Schwämmen beteiligt ist, wurde unter Anwendung der Technik des "Differential Display" der mRNA unter Benutzung des *in vitro* Primrhorphe-Zellkuttursystems (siehe unten) aufgefunden.

Die Silicase ist in der Lage, zwei Funktionen auszuüben: erstens besitzt sie die Fähigkeit (i) - in Analogie zu der Carboanhydrase - Kalkmaterial aufzulösen und (ii) - und dies war überraschend - auch Siliciumdioxid aufzulösen unter Bildung von Kieselsäure. Deshalb ist die - zuerst in S. *domuncula* aufgefundene - Silicase in der Lage, sowohl beim Katabolismus von kalkhaltigem Material als auch beim Katabolismus der kieselsäurehaltigen Spiculae mitzuwirken.

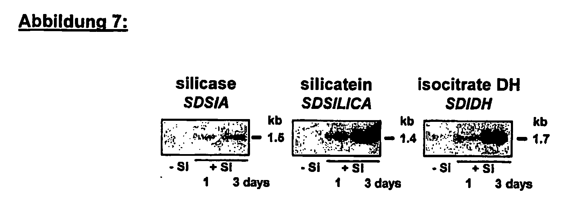

Neu an der vorliegenden Erfindung ist außerdem, daß das Silicase-Gen durch Erhöhung der Silicium-Konzentrationen im Medium (auf gewöhnlicherweise 60 µM) induziert werden kann (siehe Abbildung 7).

Gemäß einem weiteren Aspekt der vorliegenden Erfindung wird allgemein ein Verfahren zum *in vitro* oder *in vivo* Abbau von amorphem oder kristallinem Siliciumdioxid (Kondensationsprodukte der Kieselsäure, Silicate), Siliconen und anderen Silicium(IV)- oder Metalt(IV)-Verbindungen sowie von Mischpolymeren dieser Verbindungen zur Verfügung gestellt, wobei ein Polypeptid oder ein Metallkomplex eines Polypeptids zum Abbau eingesetzt wird, dadurch gekennzeichnet, daß
a) das Polypeptid eine tierische, bakterielle, pflanzliche oder Pilz Carboanhydrase-Domäne umfaßt, die mindestens 25% Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.
Bisher war es nicht bekannt, daß solche Carboanhydrasen-Domänen enthaltende Enzyme in der Lage sind, solche Silikate oder Silicone abzubauen. Aufgrund der Reversibilität dieses Prozesses betrifft ein weiterer Aspekt der vorliegenden Erfindung ein Verfahren zur Synthese von amorphem Siliciumdioxid (Kondensationsprodukte der Kieselsäure, Silicate), Siliconen und anderen Silicium(IV)- oder Metall(IV)-Verbindungen sowie von Mischpolymeren dieser Verbindungen, wobei ein Polypeptid oder ein Metallkomplex eines Polypeptids zur Synthese eingesetzt wird, dadurch gekennzeichnet, daß
a) das Polypeptid eine tierische, bakterielle, pflanzliche oder Pilz Carboanhydrase-Domäne umfaßt, die mindestens 25% Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.

Bevorzugt ist ein erfindungsgemäßes Verfahren, das dadurch gekennzeichnet ist, daß zur Synthese Verbindungen wie Kieselsäuren, Monoalkoxysilantriole, Dialkoxysilandiole, Trialkoxysilanole, Tetraalkoxysilane, Alkyl- oder Aryil-Silantriole, Alkyl- oder Aryl-Monoalkoxysilandiole, Alkyl- oder Aryl-Dialkoxysilanole, Alkyl- oder Aryl-Trialkoxysilane oder andere Metall(IV)-Verbindungen als Reaktanten (Substrate) eingesetzt werden. Durch Verwendung definierter Mischungen der Verbindungen können Mischpolymere definierter Zusammensetzung hergestellt werden.

Gemäß einem weiteren Aspekt der vorliegenden Erfindung kann durch das Polypeptid oder ein Metallkomplex des Polypeptids oder die Bindung des Polypeptids oder eines Metallkomplex des Polypeptids an andere Moleküle oder die Oberflächen von Glas, Metallen, Metalloxiden, Kunststoffen, Biopolymeren oder anderen Materialien als Template die Ausbildung definierter zwei- und dreidimensialer Strukturen erfolgen.

Gemäß einem weiteren Aspekt der vorliegenden Erfindung wird ein Verfahren zur Modifikation einer Kieselsäure oder Silicium(IV)- oder Metall(IV)-Verbindung enthaltenden Struktur oder Oberfläche zur Verfügung gestellt, wobei ein Polypeptid oder ein Metallkomplex eines Polypeptids zur Modifikation eingesetzt wird, dadurch gekennzeichnet, daß
a) das Polypeptid eine tierische, bakterielle, pflanzliche oder Pilz Carboanhydrase-Domäne umfaßt, die mindestens 25% Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.
Bevorzugterweise liegt die Kieselsäure enthaltende Struktur oder Oberfläche in Form eines Edelsteins oder Halbedelsteins vor.

Bevorzugt ist ein erfindungsgemäßes Verfahren, wobei die Modifikation eine Glättung, ein Anätzen oder ein Herstellen von Ausbohrungen der Kieselsäure oder Silicium(IV)- oder Metall(IV)-Verbindung enthaltenden Struktur oder Oberfläche durch das Polypeptid oder einen Metallkomplex des Polypeptids umfaßt.

Ein weiterer Aspekt der vorliegenden Erfindung betrifft eine chemische Verbindung oder Kieselsäure-enthaltende Struktur oder Oberfläche, die mit einem erfindungsgemäßen Verfahren erhalten wurde, insbesondere in Form eines Edelsteins oder Halbedelsteins.

Ein weiterer Aspekt der vorliegenden Erfindung betrifft auch ein Polypeptid einer Silicase aus *Suberites domuncula* gemäß SEQ ID Nr. 2 oder ein dazu homologes Polypeptid, dadurch gekennzeichnet daß
a) das Polypeptid eine tierische, bakterielle, pflanzliche oder Pilz Carboanhydrase-Domäne umfaßt, die mindestens 25% Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.
oder ein Metallkomplex davon.

Ein weiterer Aspekt der vorliegenden Erfindung betrifft auch eine Nukleinsäure, insbesondere gemäß SEQ ID Nr. 1, dadurch gekennzeichnet, daß sie im wesentlichen für ein Polypeptid der Erfindung kodiert. Die erfindungsgemäße Nukleinsäure kann in Form einer DNA, cDNA, RNA oder Gemischs davon vorliegen und dadurch gekennzeichnet sein, daß die Sequenz der Nukleinsäure mindestens ein Intron und/oder eine polyA-Sequenz aufweist. Ein anderer Aspekt der vorliegenden Erfindung betrifft die erfindungsgemäße Nukleinsäure in Form ihrer komplementären "antisense"-Sequenz.

Ein noch weiterer Aspekt der vorliegenden Erfindung betrifft auch eine erfindungsgemäße Nukleinsäure in Form eines (a) Fusionsprotein- (chimäres Protein) Konstrukts, (b) Konstrukts mit getrennter Protein-Expression (Protease-Spaltstelle) oder (c) Konstrukts mit getrennter Protein-Expression (Kassetten-Expression). Die erfindungsgemäße Nukleinsäure kann synthetisch hergestellt worden sein. Verfahren dazu sind im Stand der Technik gut bekannt.

Ein weiterer Aspekt der Erfindung betrifft einen Vektor, vorzugsweise in Form eines Plasmids, shuttle Vektors, Phagemids, Cosmids, Expressionsvektors, retroviralen Vektors, adenoviralen Vektors oder Partikels, Nanopartikeln oder Liposomen, wobei der Vektor eine erfindungsgemäße Nukleinsäure enthält. Weiterhin können Vektoren zum Transfer von Proteinen vorgesehen werden, vorzugsweise in Form eines Nanopartikels oder Liposoms, die ein Polypeptid der Erfindung umfassen.

Gemäß einem weiteren Aspekt der vorliegenden Erfindung wird eine Wirtszelle, transfiziert mit einem Vektor oder infiziert oder transduziert mit einem Partikel gemäß der Erfindung, zur Verfügung gestellt. Diese Wirtszelle nach kann dadurch gekennzeichnet sein, daß sie ein Polypeptid nach Anspruch 1, einen Metallkomplex des Polypeptids oder Teile davon exprimiert. Als Wirtzellen eignen sich alle bekannten Wirtszell-Organismen, so u.a. Hefen, Pilze, Schwämmw, Bakterien, CHO-Zellen oder Insektenzellen.

Das erfindungsgemäße Polypeptid kann dadurch gekennzeichnet sein, daß es synthetisch hergestellt worden ist oder daß das Polypeptid oder der Metallkomplex des Polypeptids in einem prokaryotischen oder eukaryotischen Zellextrakt oder -lysat vorliegt. Der Zellextrakt oder das Lysat kann aus einer Zelle ex vivo oder ex vitro gewonnen werden, zum Beispiel einer rekombinanten bakteriellen Zelle oder einem Meeresschwamm.

Das erfindungsgemäße Polypeptid kann nach herkömmlichen im Stand der Technik bekannten Verfahren gereinigt werden und somit im wesentlichen frei von anderen Proteinen vorliegen.

Ein weiterer Aspekt der vorliegenden Erfindung betrifft dann ein Verfahren zur Auffindung von Inhibitoren oder Aktivatoren eines Polypeptids einer Silicase aus *Suberites domuncula* gemäß SEQ ID Nr. 2 oder einem dazu homologen Polypeptid, das in der Aminosäuresequenz der Carboanhydrase-Domäne mindestens 25% Sequenzähnlichkeit zu der in SEQ ID Nr. 2 gezeigten Sequenz besitzt, wobei a) ein Polypeptid einer Silicase aus *Suberites domuncula* gemäß SEQ ID Nr. 2 oder ein dazu homologes Polypeptid, das in der Aminosäuresequenz der Carboanhydrase-Domäne mindestens 25% Sequenzähnlichkeit zu der in SEQ ID Nr. 2 gezeigten Sequenz besitzt zur Verfügung gestellt wird, b) das Polypeptid aus Schritt a) mit einem opotentiellen Inhibitor oder Aktivator in Kontakt gebracht wird, und c) die Fähigkeit des Polypeptids gemessen wird, Silikate oder Silicone abzubauen oder zu synthetisieren. Mit diesem Verfahren lassen sich wertvolle Substanzen aufspühren, die sich unter Umständen als Therapeutika eignen (dazu siehe im folgenden). Verfahren zur Auffindung solcher Substanzen sind dem Fachmann bekannt und schließen z.B. die Verwendung von radioaktiv markierten oder enzymatisch markierten Kandidaten-Verbindungen ein. Verfahren zur Messung der Aktivität der Silicase sind im folgenden beschrieben und können leicht durch den Fachmann auf ein Testformat hin angepaßt werden. Ein Inhibitor verringert dabei die Aktivität des Enzyms im wesentlichen vollständig, ein Aktivator induziert eine Aktivität oder verstärkt diese über den Ausgangsspiegel.

Gemäß einer Alternative des Verfahrens kann das Polypeptid einer Silicase aus *Suberites domuncula* gemäß SEQ ID Nr. 2 oder ein dazu homologes Polypeptid, das in der Aminosäuresequenz der Carboanhydrase-Domäne mindestens 25% Sequenzähnlichkeit zu der in SEQ ID Nr. 2 gezeigten Sequenz besitzt *in vivo,* in einem prokaryontischen oder eukaryontischen Zellextrakt oder -lysat oder in gereinigter Form für den Test zur Verfügung gestellt wird.

Ein noch weiterer Aspekt der Erfindung betrifft ein Verfahren zur Herstellung einer pharmazeutischen Zusammensetzung, umfassend a) Auffinden eines Inhibitors oder Aktivators nach Anspruch 25 oder 26 und b) Mischen des wie oben angegeben aufgefundenen Inhibitors oder Aktivators mit einem pharmazeutisch geeigneten Träger oder Hilfsstoff. Mittels dieser Zusammensetzung werden wertvolle Pharmazeutika zur Verfügung gestellt, die, wie das Polypeptid oder eine Nukleinsäure der Erfindung zur Prävention oder Therapie der Silikose - verwendet werden können. Bevorzugt ist eine Verwendung, wobei die Prävention und Therapie der Silikose durch Auflösen von Quarzkristallen erfolgt. Weiterhin kann die Verwendung eines Polypeptids oder einer Nukleinsäure oder pharmazeutischen Zusammensetzung gemäß der Erfindung zur Resorption oder zur Modulation der Resorbierbarkeit von Siliconen und Silicon-Implantaten erfolgen. Schließlich läßt sich die vorliegende Erfindung auch zur Transfektion von Zellen mit erfindungsgemäßen Nukleinsäuren zur Resorption oder zur Modulation der Resorbierbarkeit von Siliconen und Silicon-Implantaten verwenden. Die oben genannten Verwendungen und die Verfahren dazu sind dem Fachmann bekannt und können leicht auf die hier vorhandenen Bedürfnisse und Anforderungen angepaßt werden.

### 1.5. Klonierung des die Silicase codierenden Gens

Unter Anwendung der Technik des "Differential Display" wurde eine cDNA identifiziert, die für eine Carboanhydrase codiert. Bei Carboanhydrasen war bisher lediglich eine Beteiligung an der pH-Regulation, der HCO³-Reabsorption und der CO₂-Exspiration bekannt, nicht jedoch eine Beteiligung an der - bisher unbekannten - enzymatischen Auflösung von Siliciumdioxid-Materialien.

Die für die Silicase aus dem Meeresschwamm *S. domuncula* codierende cDNA (genannt. *SDSIA*) sowie das aus der Nucleotidsequenz abgeleitete Polypeptid (genannt: SIA_SUBDO) besitzen folgende Eigenschaften. Länge der cDNA: 1395 Nucleotide (nt); offenes Leseraster: von nt₁₂₂ - nt₁₂₄ bis nt₁₂₅₉ - nt₁₂₆₁ (Stoppcodon); Länge des Polypeptids: 379 Aminosäuren; relative Molekülmasse (Mᵣ) des Polypeptids: 43131; isoelektrischer Punkt (pl): 6,5 (berechnet mit: PC/GENE (1995) Data Banks CD-ROM; Release 14.0. IntelliGenetics, Inc. Mountain View, CA).

Die Northem-Blot-Analyse mit dem Schwamm *SDSIA*-Klon als Sonde ergibt eine Bande von ≈ 1,5 kb.

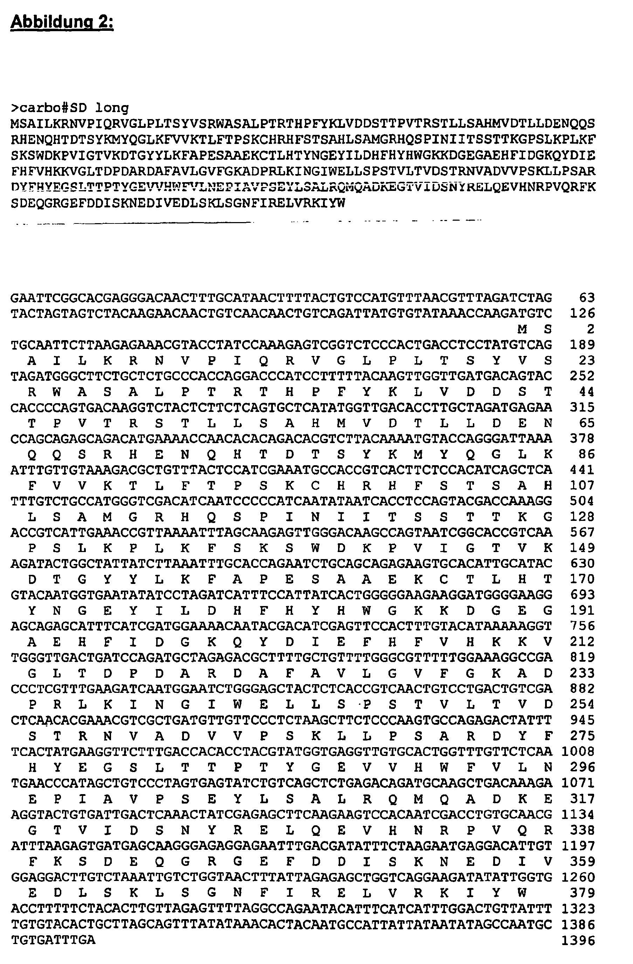

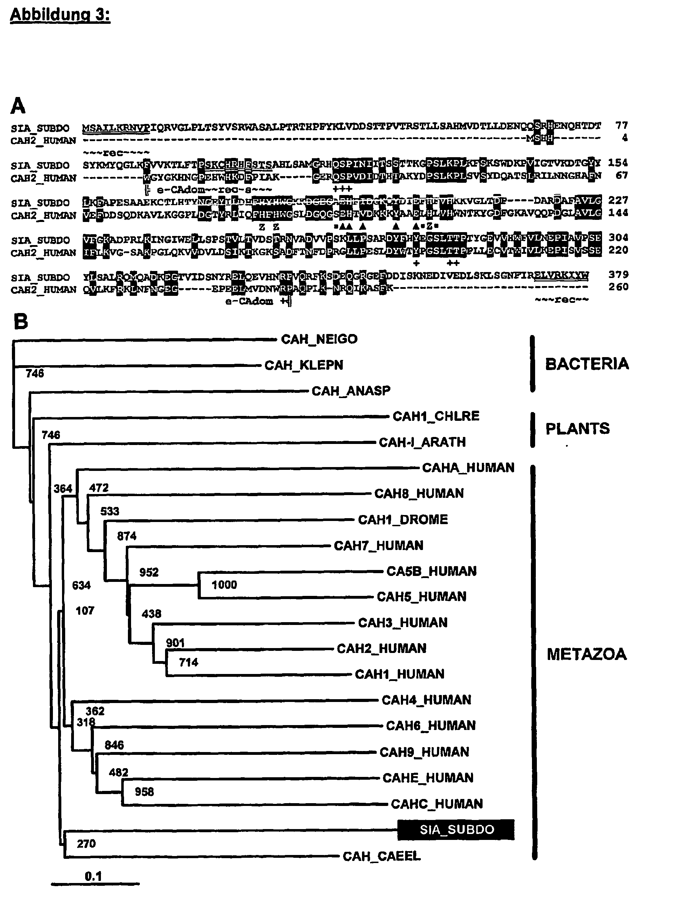

Abbildung 2 (unten) zeigt die Nucleotidsequenz der - mit Hilfe der Differential-Display-Technik identifizierten - Schwamm-Silicase-cDNA und Abbildung 2 (oben und unten) sowie Abbildung 3A zeigen das aus der Nucleotidsequenz abgeleitete Polypeptid der Schwamm-Silicase (SIA_SUBDO).

Die abgeleitete Aminosäuresequenz der Schwamm-Silicase besitzt eine große Ähnlichkeit zu den Aminosäuresequenzen der Carboanhydrase-Familie. Bis jetzt wurden mehr als sieben Isoenzyme der Carboanhydrasen bei Menschen identifiziert (Sun MK, Alkon DL (2002) Carbonic anhydrase gating of attention: memory therapy and enhancement. Trends Pharmac Sci 23:83-89). Der "Expect value" [*E*] (Coligan JE, Dunn BM, Ploegh HL, Speicher DW, Wingfield PT (2000) Current protocols in protein science. John Wiley & Sons, Chichester) der Schwamm-Silicase mit der humanen Carboanhydrases II (CAH2_HUMAN; P00918) beträgt 2e⁻²⁹. Die Eukaryonten-Typ-Carboanhydrase-Domäne (PFAM00194 [www.ncbi.nlm.nig.gov]) wird bei der Schwamm-Silicase im Aminosäure-Bereich von aa₈₇ bis aa₃₃₅ gefunden (Abbildung 3A). Das Alignment der Schwamm-Silicase mit der humanen Carboanhydrase II zeigt, daß die meisten der charakteristischen Aminosäuren, welche die Eukaryonten-Typ-Carboanhydrase-Signatur bilden (Fujikawa-Adachi K, Nishimori I, Taguchi T, Yuri K, Onishi S (1999) cDNA sequence, mRNA expression, and chromosomal localization of human carbonic anhydrase-related protein, CA-RP XI. Biochim Biophys Acta 1431:518-524; Okamoto N, Fujikawa-Adachi K, Nishimori I, Taniuchi K, Onishi S (2001) cDNA sequence of human carbonic anhydrase-related protein CA-RP X and XI in human brain. Biochim Biophys Acta 1518:311-316), auch in der Schwamm-Silicase vorhanden sind. Bei der Schwamm-Sequenz sind jedoch die Aminosäurereste 192 (Alanin), 205 (Phenylalanin) und 207 (Phenylalanin) ersetzt (Abbildung 3A).

Die Carboanhydrasen bilden eine Familie von Zinkmetall-Enzymen, die an der reversiblen Hydratation von CO₂ beteiligt sind (Sly WS, Hu PY (1995) Human carbonic anhydrases and carbonic anhydrase deficiencies. Annu. Rev. Biochem. 64:375-401). Die drei konservierten Histidinreste werden in der Silicase bei den Aminosäuren aa₁₈₁, aa₁₈₃ und aa₂₀₆ gefunden (Abbildung 3A).

### 1.6. Phylogenetische Analyse der Silicase

Abbildung 3B zeigt die Position der Schwamm-Silicase unter verschiedenen, ausgewählten Vertretern der Carboanhydrase-Familie (phylogenetischer Baum; "rooted tree" mit der bakteriellen Carboanhydrase-Sequenz von *Neisseria gonorrhoeae).* Die Schwamm-Silicase bildet mit der Carboanhydrase von *Caenorhabditis elegans* die Basis für die Carbonanhydrasen der anderen Metazoen. Die Metazoen-Enzyme sind von den Pflanzen-Enzymen und auch von den bakteriellen Enzymen getrennt.

### 2. Herstellung der Silicase

Die Silicase kann aus Geweben oder Zellen gereinigt oder rekombinant hergestellt werden.

### 2.1. Reinigung der Silicase aus natürlichen Quellen

Alle Schritte werden bei 4°C durchgeführt. Zur Reinigung der Silicase (oder Carboanhydrase oder Carboanhydrase-verwandtes Enzym) wird das - beispielsweise in einem Tris-SO₄ / Natriumsulfat-Puffer (pH 8,7) - homogenisierte Gewebe (oder werden die in diesem Puffer homogenisierten Zellen) zentrifugiert und zu dem erhaltenen Überstand eine Affinitätschromatographie-Matrix wie beispielsweise CM-Bio-Gel A, gekoppelt mit *p*-Aminomethylbenzolsulfonsäureamid, hinzugegeben. Danach wird die Suspension auf einem Rotationsschüttler (beispielsweise für 24 h) inkubiert. Das Affinitätsgel wird dann durch Absaugen über einen Glasfilter gesammelt und mit einem Puffer (beispielsweise 0,1 M Tris-SO₄, pH 8,7, enthaltend 0,2 M Na₂SO₄, 1 mM Benzamidin und 20% Glycerin) gewaschen. Danach ist es zweckmäßig, einen zweiten Waschschritt mit demselben Puffer bei einem niedrigeren pH (beispielsweise pH 7,0) anzuschließen, um unspezifisch gebundene Proteine zu entfernen. Das Gel wird dann in eine Säule überführt und mit demselben Puffer (pH 7,0) gewaschen. Zur Elution des Enzyms kann beispielweise ein 0,1 M Tris-SO₄-Puffer, pH 7,0, enthaltend 0,4 M NaN₃, 1 mM Benzamidin und 20% Glycerin, benutzt werden. Das eluierte Enzymprotein wird dann beispielsweise gegen einen 10 mM Tris-SO₄-Puffer, pH 7,5, enthaltend 1 mM Benzamidin, dialysiert und danach auf eine Ionenaustauscher-Säule (beispielsweise DEAE-Sephacel) gegeben, die beispielsweise mit 10 mM Tris-SO₄, pH 7,5, äquilibriert worden ist. Nach dem Waschen mit demselben Puffer wird das Enzym durch Anlegen eines linearen Salz-Gradienten (beispielsweise 0 bis 0,1 M Na₂SO₄) eluiert und gesammelt. Mit Hilfe dieser Prozedur kann die Silicase unter anderem aus dem Schwamm S. *domuncula* gereinigt werden.

### 2.2. Herstellung der rekombinanten Silicase

### 2.2.1. Clonierung der cDNAs aus marinen Schwämmen

Die Durchführung der Technik des "Differential Display" der mRNA/Transkripte ist Stand der Technik (Müller WEG, Krasko A, Skorokhod A, Bünz C, Grebenjuk VA, Steffen R, Batel R, Müller IM, Schröder HC (2002) Histocompatibility reaction in the sponge Suberites domuncula on tissue and cellular level: central role of the allograft inflammatory factor 1. Immunogenetics 54, 48-58). Gesamt-RNA wird von Kontrollkulturen (gehalten bei einer niedrigen Silicium-Konzentration von 5 µM) sowie von in Gegenwart von 60 µM Silicium behandelten Kulturen unter Benutzung von TRIzol Reagens (GibcoBRL) isoliert. Die Synthese des ersten cDNA-Stranges wird mit "anchored" oligo(dT)-Primern und AMV Reverser Transkriptase nach den Vorschriften des Herstellers (Promega) durchgeführt. Nach der Synthese des ersten Stranges wird die resultierende cDNA zehnfach mit H₂O verdünnt und ein aliquotes Teil davon (2 µl) der Polymerase-Ketten-Reaktion (PCR) unterworfen. Die Reaktion wird in einem Volumen von 20 µl nach Zugabe des "arbitrary" Primers 1 (5'-GTGATCGCAG-3') oder 2 (5'-CTTGATTGCC-3') sowie von 2 µM dNTP, T₁₁GC, 5 Units BioThem Polymerase (Genecraft) und [α-³²P] dATP durchgeführt. Folgende Reaktionsbedingungen haben sich bei der PCR als geeignet erwiesen: initiale Denaturierung bei 95°C für 5 Minuten, dann 40 Amplifizierungscyclen bei je 95°C für 20 Sekunden, 42°C für 120 Sekunden, 72°C für 30 Sekunden, gefolgt von einer Endinkubation von 10 Minuten bei 72°C. Die Proben werden dann in einem 5%igen Polyacrylamidgel (in 1 x TBE) aufgetrennt. Nach dem Lauf wird das Gel getrocknet und 4 Tage mit einem Röntgenfilm exponiert. Die interessierenden, im Autoradiogramm detektierten Banden werden ausgeschnitten, 15 Minuten in 200 µl H₂O gekocht, auf Eis gekühlt und 10 Minuten bei 14000 x g zentrifugiert. Die resultierenden Überstände werden mit dem gleichen Volumen an 10 M Ammoniumacetat, 20 µg/ml tRNA versetzt und mit 2,5 Volumina an Ethanol bei -80°C über Nacht präzipitiert. Die cDNA-Pellets werden dreimal in 75% Ethanol gewaschen und in 20 µl H₂O gelöst.

Etwa 2 µl der eluierten Banden werden in 50 µl-Reaktionsansätzen unter Benutzung der oben beschriebenen Primer unter denselben Bedingungen reamplifiziert, in einem pGEM-T-Vector (Promega) subcloniert und sequenziert.

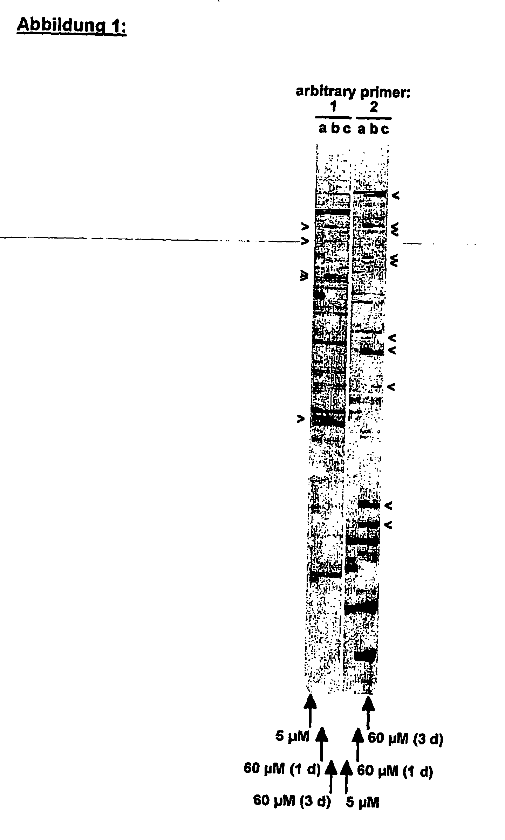

Diejenigen Transkripte werden ausgewählt, die differentiell exprimiert werden, d. h. die zusätzlich in den Gelen mit den RNAs von Zellen erhalten werden, die mit 60 µM Silica behandelt wurden (Abbildung 1). Die identifizierten cDNAs/Transkripte werden mit den in der BLAST Data Base enthaltenen Sequenzen verglichen. In dem in Abbildung 1 gegebenen Beispiel zeigten die folgenden Moleküle die größte Verwandtschaft: Calcium/Calmodulin-abhängige Proteinkinase (CaM Kinase) II gamma (XM_044349; Expect value [*E*]: 1e⁻¹⁶); hypothetisches Protein (XP_101359, *E* 1,6); MUC3B Mucin (AJ291390, *E* 0,20); hypothetisches Protein (XP_067115, *E* 5,9); hypothetisches Protein (XP_090138, *E* 2,9); ATP-bindende Kassette, Unterfamilie A Mitglied 4 (XM_001290, *E* 1,6); Polypeptid ähnlich dem Zinkfingerprotein 91 (XM_091947, *E* 3,1); hypothetisches Protein (XP_104250, *E* 0,48), hypothetisches Protein (XP_169372, *E* 8,6); hypothetisches Protein (XP_104250, *E* 4,1), hypothetisches Protein (XP_098020, *E* 3,3) und hypothetisches Protein (XP_169372, *E* 8,6).

Zusätzlich zu diesen Sequenzen wurde die Silicase als weiteres Transkript identifiziert und in größerem Detail analysiert.

Das Silicase-Gen kann auch aus cDNA-Bibliotheken, z. B. in *ZapExpress* und in *Escherichia coli* XL1-Blue MRF', mit geeigneten degenerierten Primem mittels der PCR-Technik identifiziert werden; hierzu werden entsprechende vektorspezifische Primer eingesetzt. Die erhaltenen Syntheseprodukte werden zum Screenen in den betreffenden cDNA-Bibliotheken benutzt. Danach werden die identifizierten Clone in einen Vektor (beispielsweise pGem-T) subcloniert und anschließend sequenziert.

### 2.2.2. Expression und Isolierung der rekombinanten Silicase

Die Herstellung der rekombinanten Silicase (genannt: rSIA_SUBDO) erfolgt bevorzugt in *E. coli.* Aber auch die Herstellung in Hefen und Säugerzellen ist möglich und wurde erfolgreich durchgeführt. Im Folgenden ist als Beispiel die Expression des *SDSIA*-Gens von *S*. *domuncula* in *E*. *coli* unter Benutzung des "GST (Glutathion-S-Transferase) Fusions"-Systems (Amersham) beschrieben. In dem Beispiel werden zwei Inserte benutzt, um potentielle Effekte von Signalpeptiden während der Expression zu eliminieren; ein Insert umfaßt das gesamte abgeleitete Protein (lange Form; von der Aminosäure aa₁ bis zur Aminosäure aa₃₇₉) und das andere Insert lediglich die Aminosäuren aa₉₆ bis aa₃₇₉ (kurze Form) (Abbildung 3A). Die entsprechenden Klone werden als *SDSIA-I* und *SDSIA-s* bezeichnet. Sie werden in einen entsprechenden Vektor eincloniert, z. B. in das Plasmid *pGEX-4T-2,* welches das Glutathion S-Transferase (GST)-Gen von *Schistosoma japonicum* enthält. Auch andere Expressionsvektoren haben sich als geeignet erwiesen. Nach Transformation von *E*. *coli* wird die Expression der Silicase üblicherweise durch IPTG (Isopropyl-β-D-thiogalactopyranosid) induziert und in Gegenwart von 1 mM ZnSO₄ für 4 oder 6 Stunden bei 37°C durchgeführt (Ausubel FM, Brent R, Kingston RE, Moore DD, Smith JA, Seidmann JG, Struhl K (1995) Current Protocols in Molecular Biology. John Wiley and Sons, New York). Die erhaltenen GST-Fusionsproteine mit der Bezeichnung rSIA_SUBDO-I (lange Form; Mr 69 kDa) bzw. rSIA_SUBDO-s (kurze Form; Mᵣ 58 kDa) werden z. B. durch Affinitätschromatographie an Glutathion-Sepharose 4B gereinigt. Zur Abtrennung der Glutathion-S-transferase von der rekombinanten Schwamm-Silicase werden die Fusionsproteine mit Thrombin (10 Units/mg) gespalten. Die Proteine werden dann der Gelelektrophorese in Gegenwart von 2-Mercaptoethanol unterworfen. Die Gelelektrophorese kann in 10%igen Polyacrylamidgelen mit 0,1% NaDodSO₄ (PAGE) durchgeführt werden. Die Gele werden mit Coomassie Brillant Blau gefärbt

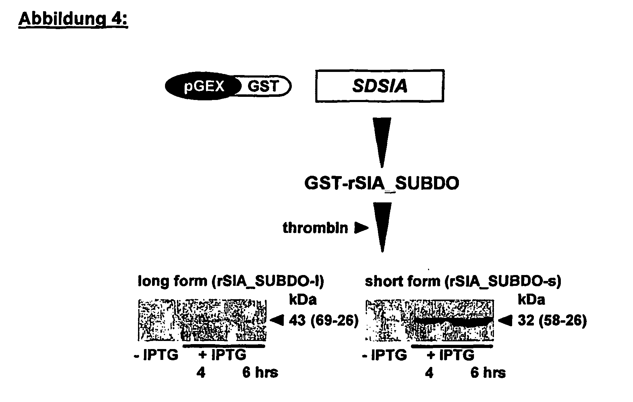

Nach der Spaltung, Reinigung und anschließender PAGE werden die lange Form (rSIA_SUBDO-I [43 kDa]) und die kurze Form (rSIA-SUBDO-s [32 kDa]) der rekombinanten Proteine erhalten (Abbildung 4).

### 2.2.3. Expression und Isolierung der rekombinanten Silicase aus weiteren Organismen

Entsprechend der oben beschriebenen Vorgehensweise kann die Isolierung, Clonierung und Expression der Silicase-cDNA auch aus anderen Organismen durchgeführt werden, beispielsweise aus (Siliciumdioxid-produzierenden) Diatomeen (z. B. *Cylindrotheca fusiformis).* Die Gewinnung von Diatomeen in axenischen Kulturen ist Stand der Technik (Kröger N, Bergsdorf C, Sumper M (1996) Europ J Biochem 239:259-264).

### 2. 3. Isolierung und Reinigung der Silicase mittels Antikörper

Nach Extraktion oder partieller Renigung nach einem der oben geschilderten Verfahren wird die Silicase an einer Antikörper-Affinitätsmatrix gereinigt. Die Affinitätsmatrix wird hergestellt, indem ein Silicase-spezifischer Antikörper an eine feste Phase (CNBr-aktivierte Sepharose oder anderer geeigneter Träger) immobilisiert wird. Als Antikörper werden monoklonale oder polyklonale Antikörper gegen das Silicase eingesetzt, die nach Standard-Methoden hergestellt werden (Osterman LA (1984) Methods of Protein and Nucleic Acid Research, Vol 2, Springer-Verlag, Berlin). Die Kopplung des Antikörpers an die Säulenmatrix wird nach den Angaben des Herstellers (Pharmacia) durchgeführt. Die Elution der reinen Silicase erfolgt mittels pH-Änderung oder Änderung der Ionenstärke.

### 3. Nachweis der Silicase-Aktivität

Im Folgenden werden lediglich die Aktivitäten, die für die kurze Form der rekombinanten Schwamm-Silicase (rSIA_SUBDO-s) gefunden werden, angegeben.

### 3.1. Carboanhydrase-Aktivität

Zur Bestimmung der Carboanhydrase-Aktivität der rSIA_SUBDO-s kann ein Assay angewandt werden, der auf der Hydrolyse von *p*-Nitrophenylacetat basiert (Armstrong JM, Myers DV, Verpoorte JA, Edsall JT (1966) Purification and properties of human erythrocyte carbonic anhydrase. J Biol Chem 241:5137-5149). 0,5 ml einer 3 mM *p*-Nitrophenylacetat-Lösung (Sigma) wird mit 0,05 ml eines 0,3 mM Tris-HCl-Puffers (pH 7,6) gemischt. Nach Präinkubation bei 25°C für 5 Minuten werden 50 µl der rekombinanten Silicase (rSIA_SUBDO) hinzugefügt, und der Anstieg der Extinktion bei 348 nm wird über einen Zeitraum von 5 Minuten bestimmt.

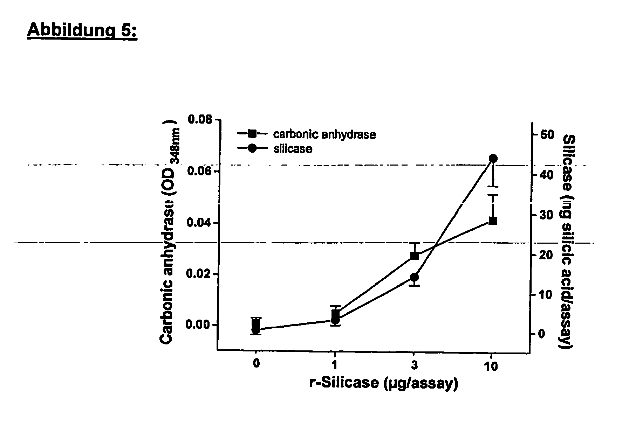

Abbildung 5 zeigt, daß die Aktivität der rekombinanten Silicase von der Konzentration des Enzyms im Assay abhängt. Die Enzymaktivität wird in optische Dichte (OD)-Einheiten pro Minute angegeben. Der Zusatz von 1 µg Silicase pro Assay (0,56 µl) ergab eine Aktivität von 0,005 OD₃₄₈ₙₘ, die mit steigender Proteinkonzentration bis auf 0,04 OD₃₄₈ₙₘ anstieg.

### 3.2. Silicase-Aktivität

Als Substrat (amorphes Siliciumdioxid) für die Silicase können beispielsweise Spiculae von *S. domuncula* dienen. Die Spiculae können aus Schwammgewebe durch 12-stündige Inkubation in Gegenwart von Ethylendiamintetraessigsäure (20 mM, in PBS; PBS = Phosphatpuffer-Salz-Lösung, bestehend aus 1,15 mM KH₂PO₄, 8,1 mM Na₂HPO₄, 137 mM NaCl und 2,7 mM KCI) erhalten werden. Nach Waschen mit destilliertem Wasser und mit Ethanol (zweimal) werden die Spiculae getrocknet (56°C) und dann in einem Mörser zu einem Pulver zerrieben.

Die Silicase-Aktivität kann wie folgt bestimmt werden. Üblicherweise werden 100 µg der getrockneten Spiculae (Pulver) zu einem geeigneten Puffer wie 50 mM Tris-HCl-Puffer (pH 7,2; 10 mM DL-Dithiothreitol, 100 mM NaCl) und 0,5 mM ZnSO₄ in 2 ml Eppendorf-Gefäßen hinzugegeben. Dann werden üblicherweise 50 µl der rekombinanten Silicase hinzugefügt und bei 25°C inkubiert (die Inkubation ist auch bei anderen Temperaturen zwischen 5°C und etwa 65°C möglich). Die durchschnittliche Inkubationszeit beträgt 60 Minuten. Zur quantitativen Bestimmung der Menge an gelöstem Siliciumdioxid werden die nichtgelösten Spiculae abzentrifugiert (14000 x g; 15 Minuten; 4°C). Die freigesetzte, lösliche Kieselsäure kann z. B. mit Hilfe eines Molybdat-gestützten Nachweisverfahrens, wie z. B. dem kolorimetrischen "Silicon Test" (Merck; 1.14794), quantitativ bestimmt werden. Die Menge an Kieselsäure wird in diesem Fall anhand einer Kalibrierungskurve mit einem Siliciumstandard (Merck 1.09947) aus den Extinktionswerten bei 810 nm berechnet.

Abbildung 5 zeigt, daß die rekombinante Silicase den Abbau (Auflösung) von amorphem Siliciumdioxid katalysiert. Üblicherweise werden bei einer EnzymKonzentration von 1 µg rekombinante Silicase pro Assay 3 ng Kieselsäure/Assay freigesetzt. Bei höheren Proteinkonzentrationen (3 oder 10 µg/Assay) beträgt die Freisetzung von Kieselsäure 20 bzw. 43 ng/Assay.

### 3.3. Silicase-Aktivität in Escherichia coli-Lysat

Die Silicase-Aktivität ist auch in Lysaten von *E. coli* nachweisbar, die mit dem *SDSIA-*Gens von S. *domuncula* (in dem folgenden Beispiel wurde die kurze Form verwendet; = *SDSIA-s*) unter Benutzung des "GST Fusions"-Systems transformiert wurden. Bei dem in Tabelle 1 gezeigten Experiment wurden Schwamm-Spiculae (Nadeln; 1 mg) mit 1,5 ml Lysat, dem 1 mM ZnCl₂ und 0,1 M NaCl hinzugefügt wurden, bei verschiedenen Temperaturen inkubiert. Nach 1, 3, 6 und 24 h wurden die Proben durch Erhitzen auf 95°C für 10 min denaturiert (zur Inaktivierung der Silicase) und dann mit Proteinase K (30 Units/ml) bei 37°C für 1 h inkubiert. Danach wurde zentrifugiert (5 min, 14000 rpm) und der Molybdat-Assay (Kit der Firma Merck; siehe oben) zur Bestimmung des freigesetzten Silicats durchgeführt. Es wurde gefunden, daß bei 4°C nur eine sehr geringfügige Menge an Silicat freigesetzt wurde, bei Raumtemperatur (22°C) und 56°C jedoch bis zu 3,4 bzw. 4,1 ng/rnl (24 h).

Eine etwas geringere Menge an freigesetztem Silicat ließ sich auch in Lysaten aus nichttransformierten *E*. *coli* nachweisen, was zeigt, daß auch in bakteriellen Zellextrakten eine deutliche Silicat-abbauende Aktivität vorhanden ist.

**Tabelle 1. Silicase-Aktivität in Lysaten von transformierten (+) und nichttransformierten (-) E. coli bei verschiedenen Inkubationstemperaturen. Zur Transformation wurde die kurze Form des SDSIA-Gens von S. domuncula (= SDSIA-s) verwendet. Die Freisetzung von Silicat wurde nach einer Inkubationszeit von 1, 3, 6 und 24 Stunden bestimmt.**

| Temperatur | Freigesetztes Silicat (ng/ml) | | | |
|---|---|---|---|---|
| | 1h | 3h | 6h | 24h |
| 4°C (-) | 0,113 | 0,124 | 0,242 | 0,303 |
| 4°C (+) | 0,110 | 0,140 | 0,526 | 0,828 |
| 22°C (-) | 0,197 | 0,415 | 1,467 | 2,068 |
| 22°C (+) | 0,528 | 0,540 | 1,939 | 3,409 |
| 56°C (-) | 0,345 | 1,009 | 1,824 | 2,447 |
| 56°C (+) | 1,542 | 1,747 | 2,275 | 4,092 |

### 3.4. Silicase-Aktivität kommerzieller Carboanhydrasen

Silicase-Aktivität ist nicht nur bei dem Schwammenzym, sondern überraschenderweise auch bei kommerziellen Carboanhydrasen meßbar. Tabelle 2 zeigt die Freisetzung von Silicat aus Diatomeen-Skeletten (Silicat-Gerüsten von Kieselalgen) sowie aus Sand durch eine kommerzielle Carboanhydrase-Präparation (aus Rinder-Erythrozyten; Firma Calbiochem). In dem Experiment wurden die Silicat-Proben zunächst zweimal mit Wasser und zweimal mit Äthanol gewaschen und danach getrocknet. Anschließend wurden die Proben in 50 mM Tris-HCl-Puffer, pH 7,6, suspendiert (1 mg/ml) und in 2 ml-Eppendorf-Reaktionsgefäße verteilt (100 µl pro Reaktionsgefäß; = 100 µg Silicat pro Reaktionsgefäß). Danach wurde 1,4 ml bovine Carboanhydrase (10 Units; Firma Calbiochem) in 50 mM Tris-HCl-Puffer, pH 7,6 (mit und ohne 1 mM ZnCl₂) pro Reaktionsgefäß hinzugefügt. Die Gefäße wurden bei Raumtemperatur (22°C) unter Schütteln für 24 h inkubiert. Danach wurden die Ansätze zentrifugiert (14000xg, 15 min, 4°C). Der Silicat-Gehalt im Überstand wurde mit Hilfe des Molybdat-Assay der Firma Merck (siehe oben) bestimmt.

**Tabelle 2. Silicase-Aktivität einer kommerziellen Carboanhydrase-Präparation (Firma Calbiöchem) mit und ohne Zusatz von ZnCl₂. Die Freisetzung von Silicat wurde nach einer Inkubationszeit von 24 Stunden bestimmt.**

| | Freigesetztes Silicat (ng/ml) | |
|---|---|---|
| | Diatomeen | Sand |
| Minus ZnCl₂ | 0,0018 | 0,0036 |
| Plus ZnCl₂ | 0,0233 | 0,0359 |

### 3.5. Reversiblität der Silicase-Aktivität

Die Silicase-Reaktion ist prinzipiell reversibel. Deshalb kann die Reaktion auch zur Synthese von amorphem Siliciumdioxid oder Siliconen verwendet werden. Für die Silicase-vermittelten Synthese können auch Alkyl- oder Aryl-substituierte Alkoxyverbindungen von Silicium(IV) wie Tetraalkoxysilane, Trialkoxysilanole, Dialkoxysilandiole, Monoalkoxysilantriole, Alkyl- oder Aryl-Trialkoxysilane, Alkyl- oder Aryl-Dialkoxysilanole oder Alkyl- oder Aryl-Monoalkoxysilandiole eingesetzt werden. Auch Mischungen dieser Substrate werden polymerisiert. Somit können auch Mischpolymere hergestellt werden.

### 4. Ligation der cDNA für Silicase mit einer oder mehreren cDNA(s) für anderer Proteine

### 4.1. Herstellung von Silicase-Fusionsproteinen

Zur Herstellung von Fusionsproteinen mit der Silicase wird ein geeigneter Expressionsvektor (beispielsweise *pQE-30*-Vektor; Qiagen) eingesetzt. Die Silicase-cDNA - mit z. B. einer *Bam*HI-Restriktionsstelle am 5'-Terminus und z. B. einer *Sal*I-Restriktionsstelle am 3'-Terminus - wird hergestellt. Das Stopp-Codon in der Silicase-cDNA wird entfernt. Hierzu wird die PCR-Technik eingesetzt und zum Amplifizieren Primer, welche die betreffenden Restriktionsstellen besitzen, benutzt. Die cDNA für das zweite Protein wird entsprechend gewonnen, wobei am 5'-Terminus die gleiche Schnittstelle wie am 3'-Terminus der Silicase-cDNA (im Beispiel Sa/l) und am 3'-Terminus eine von den anderen verschiedene (z. B. eine *Hin*dIII-Stelle) vorliegt. Falls sich in den betreffenden cDNAs interne Restriktionsstellen befinden, können alternative Restriktionsenzyme eingesetzt werden. Darüber hinaus können auch Linker zwischen die beiden cDNAs eingesetzt werden.

Die beiden cDNAs werden nach den üblichen Verfahren ligiert, gereinigt und in den *p*QE-30-Vektor einligiert. Die Ligation erfolgt im Anschluß an das Histidin-Tag (etwa 6 Histidin-Codons). Die Expression und Reinigung des Fusionsproteins über z. B. das Histidin-Tag, das an dem rekombinanten Protein vorliegt, kann an entsprechenden Affinitätssäulen, z. B. einer Ni-NTA-Matrix, durchgeführt werden (Skorokhod A, Schäcke H, Diehl-Seifert B, Steffen R, Hofmeister A, Müller WEG (1997) Cell Mol Biol 43:509-519).

### 4.2. Getrennte Expression I (Protease-Spaltstelle)

Alternativ zu dem Verfahren unter 4.1. kann zwischen der cDNA für die Silicase und der cDNA für ein weiteres Protein eine Protease-Spaltstelle (wie z. B. eine Enterokinase-Stelle) eincloniert werden. In diesem Falle kann ein Codon für ein neues Start-Methionin vor den codierenden Bereich des Gens für das weitere Protein insertiert werden. Nach Expression und Reinigung wird das Fusionsprotein proteolytisch gespalten. Jetzt liegen beide Proteine separat vor.

### 4.3. Getrennte Expression II (Kassetten-Expression)

Alternativ können beide Proteine auf einem Konstrukt separat exprimiert werden. Hierzu wird in einem Expressions-Vektor das Silicase-Gen dem His-Tag nachgeschaltet. Am Ende der Silicase-cDNA wir ein Stopp-Codon insertiert. Zwischen der cDNA für die Silicase und der cDNA für das weitere Protein wird eine Ribosomen-Bindungsstelle mit Codon für ein Start-Methionin eincloniert. Wiederum wird ein His-Tag der cDNA für das weitere Protein vorgeschaltet. Ebenfalls erhält dieses Gen ein Stopp-Codon.

Die His-Tags können deletiert werden, wenn die Proteine zur Funktionsanalyse in den betreffenden Wirtszellen benutzt werden.

### 4.4. Erweiterungen

Für die unter 4.1 bis 4.3 beschriebene Expression können sowohl bakterielle als auch eukaryotische Zellen benutzt werden.

Die unter 4.1 bis 4.3 beschriebene Expression kann auch für drei und mehr offene Leserahmen eingesetzt werden.

### 5. Das Modellsystem für die Synthese/den Abbau von biogenem Siliciumdioxid: Primmorphe

### 5.1. Primmorphe

Das Primmorphe-System wurde zum Patent angemeldet (DE 19824384**.** Herstellung von Primmorphe aus dissoziierten Zellen von Schwämmen, Korallen und weiteren Invertebraten: Verfahren zur Kultivierung von Zellen von Schwämmen und weiteren Invertebraten zur Produktion und Detektion von bioaktiven Substanzen, zur Detektion von Umweltgiften und zur Kultivierung dieser Tiere in Aquarien und im Freiland. Erfinder und Anmelder: Müller WEG, Brümmer F).

Primmorphe sind Aggregate, die aus proliferierenden und differenzierenden Zellen bestehen (Müller WEG, Wiens M, Batel R, Steffen R, Borojevic R, Custodio MR (1999) Establishment of a primary cell culture from a sponge: Primmorphs from Suberites domuncula. Marine Ecol Progr Ser 178:205-219). Primmorphe werden aus Schwamm-Einzelzellen, die aus Schwammgewebe nach Dissoziation in Ca²⁺- und Mg²⁺-freiem, EDTA enthaltendem künstlichem Seewasser erhalten werden, gebildet. Aus den Schwamm-Einzelzellen bilden sich nach Überführung in Ca²⁺- und Mg²⁺-enthaltendes Seewasser Aggregate, die nach 3 Tagen eine Größe von 1 mm erreichen, und nach 5 Tagen Primmorphe mit einem Durchmesser von etwa 5 mm.

Die Primmorphe werden von epithelartigen Zellen, den Pinacocyten, umgeben. Die Zellen innerhalb der Primmorphe sind überwiegend sphärulöse Zellen, daneben kommen Amoebocyten und Archaeocyten vor.

### 5.2. Effekt von Silicium auf die Spiculae-Bildung

Das Primmorphe-System von Schwämmen, z. B. *S. domuncula,* kann zur Untersuchung der Spiculae-Bildung oder -Auflösung benutzt werden.

Dazu werden Primmorphe für 8 Tage in Seewasser, das mit 30 µM Fe(+++) (hinzugegegeben ais Citrat) und 10% RPMI1640-Medium ergänzt wird, kultiviert. Die Silicium-Konzentration im Seewasser/Medium ist 5 uM. Nach 8 Tagen werden die Primmorphe entweder in diesem Medium weiter inkubiert oder in ein Medium, das 60 µM Silicium enthält (die für die Spiculae-Bildung optimale Silicium-Konzentration; hinzugegeben als Na-Hexafluorosilicat), überführt und für 1 oder 3 Tage kultiviert.

Primmorphe, die ohne Zugabe von Silicium kultiviert werden, zeigen überwiegend eine runde, sphärische Gestalt.

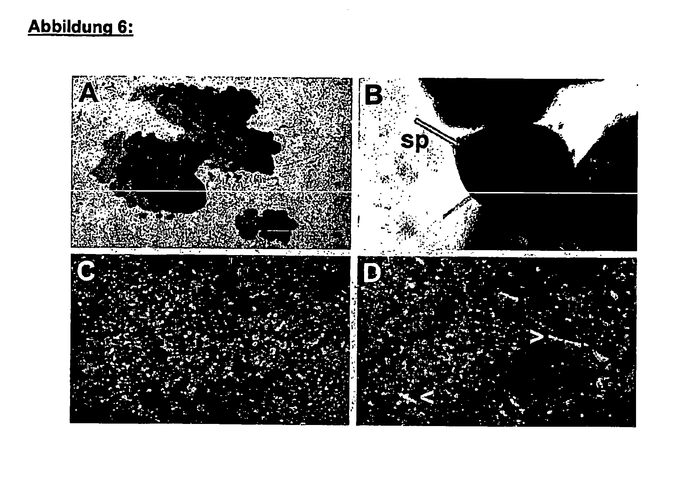

Abbildung 6A zeigt, daß die meisten der Primmorphe nach zusätzlicher 3-tägiger Kultivierung in Gegenwart von 60 µM Silicium jedoch ovalförmig werden. In Gegenwart von Silicium beginnen die Primmorphe mit der Spiculae-Bildung. Teilweise kann die Synthese von langen (>100 µm) Spiculae beobachtet werden (Abbildung 6B), meist werden jedoch kleinere Spiculae (30 µm) gefunden (Abbildung 6D). In Abwesenheit von Silicium sind keine Spiculae vorhanden (Abbildung 6C).

### 5.3. Silicium-responsive Gene

In Primmorphen von S. *domuncula* wird in Gegenwart von Silicium die Expression des Silicase-Gens hochreguliert. Daneben wird auch die Expression der folgenden Gene erhöht: Silicatein, Kollagen, Myotrophin und Isocitrat-Dehydrogenase.

Die Expression des Silicase-Gens kann durch Northern-Blotting unter Anwendung von Methoden, die Stand der Technik sind und beispielsweise zur Bestimmung der Expression von Silicatein und Kollagen angewandt wurden, bestimmt werden (Krasko A, Batel R, Schröder HC, Müller IM, Müller WEG (2000) Expression of silicatein and collagen genes in the marine sponge Suberites domuncula is controlled by silicate and myotrophin. Europ J Biochem 267:4878-4887).

In dem in Abbildung 7 gezeigten Experiment blieben die Primmorphe entweder unbehandelt oder wurden mit 60 µM Silicium für 1 bis 3 Tage inkubiert. Die RNA wurde dann extrahiert. Eine Menge von je 5 µg Gesamt-RNA wurde elektrophoretisch an einem 1%igen Formaldehyd/Agarose-Gel aufgetrennt und auf eine Hybond-N⁺ Ny-Ion-Membran entsprechend der Vorschrift des Herstellers (Amersham) geblottet. Die Hybridisierung wurde mit 400 bis 600 bp großen Segmenten der folgenden Sonden durchgeführt: *SDSIA* (codiert für Silicase), *SDSILICA* (codiert Silicatein) und *SDIDH* (codiert für die α-Untereinheit der Isocitrat-Dehydrogenase). Die Sonden wurden mit dem PCR-DIG-Probe-Synthesis Kit entsprechend der Vorschrift des Herstellers (Roche) markiert. Nach dem Waschen wurde die DIG-markierte Nucleinsäure mit anti-DIG Fab Fragmenten (konjugiert mit alkalischer Phosphatase; Verdünnung: 1:10000) detektiert und mit Hilfe der Chemolumineszenztechnik unter Verwendung von CDP (Roche), dem Chemolumineszenzsubstrat der alkalischen Phosphatase, sichtbar gemacht.

Abbildung 7 zeigt die erhaltenen Northem-Blots. Es ist sichtbar, daß die Gene für die Silicase und Silicatein als Antwort auf höhere Siliciumkonzentrationen stark hochreguliert werden. Weiterhin wird auch das Isocftrat-Dehydrogenase-Gen (codiert für ein am Citratcyclus beteiligtes Enzym) hochreguliert, was anzeigt, daß die Bildung von amorphem Siliciumdioxid eine erhöhte metabole Rate der Zellen erfordert.

### 6. Wirkmechanismus der Silicase

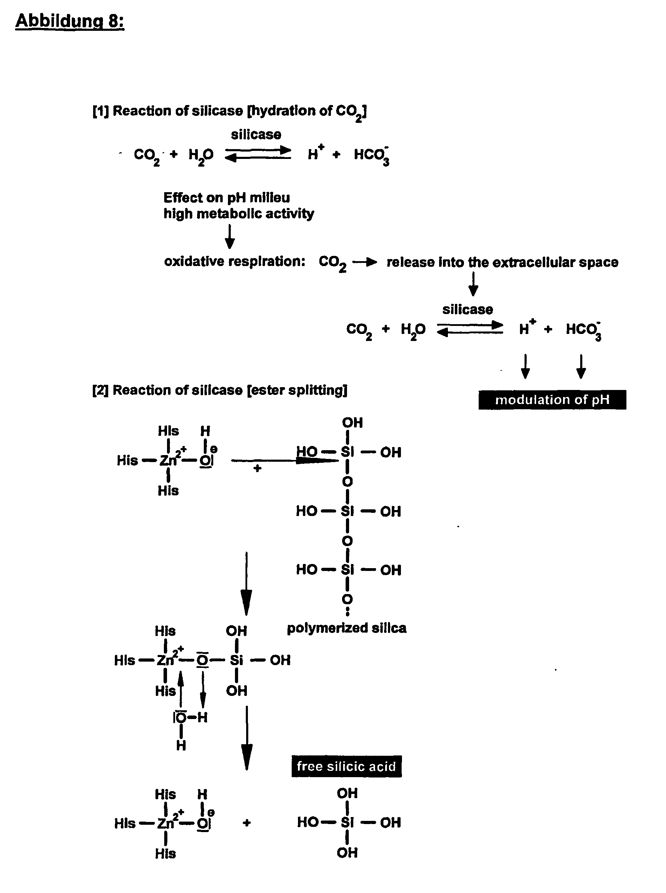

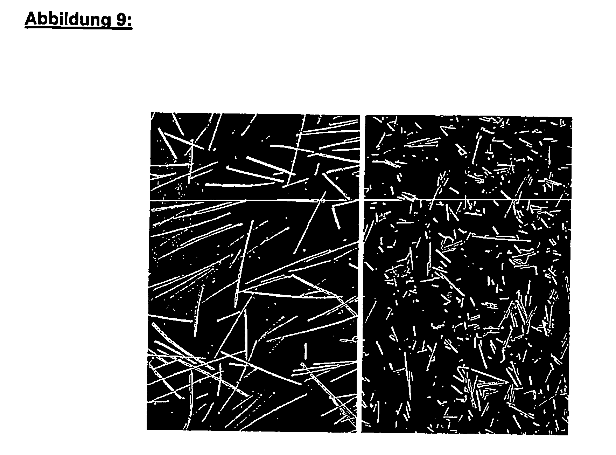

überraschend war der durch Sequenzvergleiche erhaltenen Befund, daß die Silicase ein Mitglied der Familie der Carboanhydrasen (Carbonat-Hydrolase; EC 4.2.1.1) ist. Diese Enzyme katalysieren die reversible Hydration von Kohlendioxid (Abbildung 8 [1]). Kohlendioxid wird durch die Carboanhydrase in HCO³⁻ und H⁺ umgewandelt.

Die Silicase besitzt in der Tat auch eine Carbonanhydrase-Aktivität, wie mit einem kolorimetrischen Assay (Armstrong JM, Myers DV, Verpoorte JA, Edsall JT (1966) Purification and properties of human erythrocyte carbonic anhydrase. J Biol Chem 241:5137-5149) nachgewiesen werden kann. Demzufolge ist es möglich, daß die Silicase infolge der Umwandlung von CO₂ zu HCO³⁻ eine Änderung des pH bewirkt (Abbildung 8 [1]). Dies ermöglicht eine Anätzung von Kalksubstraten, aber nicht von Siliciumdioxid-Materialien, deren Löslichkeit mit steigendem, aber nicht mit fallendem pH ansteigt.

Es ist bekannt, daß einige Schwammspezies wie Spezies des Genus *Cliona* fähig sind, Caiciumcarbonat aufzulösen und Löcher in Calcit/Aragonit-Substrate zu boren (Rützler K, Rieger G (1973) Sponge bürrowing : fine structure of Cliona lampa penetrating calcareous substrata. Mar Biol 21:144-162).

Unbekannt und überraschend war jedoch die Silicase-Aktivität des Enzyms.

Es ist bekannt, daß am Wirkmechanismus (Carboanhydrase-Aktivität) der Carboanhydrasen drei Histidinreste beteiligt sind, die an ein divalentes Zinkion binden; dementsprechend kann für die Silicase-Aktivität folgender Wirkmechanismus formuliert werden (Abbildung 8 [2]). Bei der Silicase von *S. domuncula* werden die Histidinreste in dem abgeleiteten Polypeptid bei den Aminosäurepositionen aa₁₈₁, aa₁₈₃ und aa₂₀₆ gefunden (Abbildung 3A). In Wasser (Lewis-Base) wird ein an das Zn²⁺ (Lewis-Säure) gebundenes Hydroxidanion gebildet. Dieses führt einen nucleophilen Angriff an einem der Siliciumatome durch, die über Sauerstoffatome miteinander verknüpft sind (Abbildung 8). Im nächsten Schritt bindet der Zink-Komplex an das Siliciumatom unter Spaltung der Sauerstoffbindung. Unter Verbrauch von H₂O wird letztendlich eine freie Kieselsäure freigesetzt und das initiale Zink(II)-gebundene Hydroxidanion wieder gebildet.

### 7. Verwendungen der Silicase und Silicase-Fusionsproteine

**Für die rekombinante Silicase, die aus verschiedenen Quellen gereinigte Silicase und die Silicase-Fusionsproteine ergeben sich eine Reihe unterschiedlicher industriell-technischer Verwendungen, und zwar:**
1.) Verwendung zur Oberflächenmodifikation von Biomaterialien (Verbesserung der Biokompatibilität). Oberflächen-modifizierte Biomaterialien finden u. a. Verwendung zur Beeinflussung von Zelladhäsion und Wachstum, zur Modifizierung der Blut-Kompatibilität oder zur Kontrolle der Protein-Adsorption (z. B. Herabsetzung der Adsorption von Kontaktlinsen). Eine Literatur-Übersicht findet sich in: Ratner BD et al (Hrsg) Biomaterials Science - An Introduction to Materials in Medicine. Academic Press, San Diego, 1996. Ein Problem besteht darin, daß die zur Herstellung dieser Modifikationen angewandten Bedingungen oft einen schädlichen (destruierenden) Effekt auf die benutzten Biomaterialien haben. Eine im Vergleich zu den benutzten physikalisch/chemischen Verfahren "milde", Biomaterialien schonenden Methode stellt eine Modifikation der Oberflächen dar, die allein auf biochemisch/enzymatischen Reaktionen beruht, was mit Hilfe des erfindungsgemäßen Verfahrens möglicht wird (Silicase-vermittelter enzymatischer Abbau und - als reversible Reaktion - enzymatische Synthese von SiO₂- oder Siloxan-enhaltenden Oberflächen mit Hilfe der rekombinanten/gereinigten Silicase). Insbesondere ergibt sich auch eine Verwendung der rekombinanten oder aus natürlichen Quellen gereinigten Silicase bei der Herstellung von Oberflächen-Modifikationen (beim Coating) von Silicon-Materialien, wie Silicon-Brust-Implantaten, Endoprothesen oder Metall-Implantaten (Verbesserung der Verbindung zwischen Knochen und Metall-Implantat, Biologisierung der Metall-Implantate) sowie Kontakt/Plastiklinsen. Weitere Verwendungen betreffen das Coating von Collagen, das als Knochenersatzmaterial dient, und von Collagen-Vliesen, die z. B. für das "Tissue Engineering" benutzt werden. Das Ziel ist hierbei die Erhöhung der Stabilität und der Porosität sowie die Verbesserung der Resorbierbarkeit.
2.) Verwendung zur Herstellung neuer Biomaterialien wie Knochenersatzmaterialien oder Zahnersatzmaterialien durch Co-Synthese von Polysilicaten, Siliconen oder Mischpolymeren.
3.) Verwendung zur Oberflächen-Modifikation (Kontaktzonen-Behandlung) von (Silicium)-Halbleitern oder Silicium-Chips.
4.) Verwendung zur Modifikation oder zur Synthese von Nano-Strukturen aus amorphem Siliciumdioxid. Mittels der rekombinanten Silicase, der rekombinanten Silicase-Fusionsproteine oder der gereinigten Silicase ist es möglich, spezifische zwei- und dreidimensionale Strukturen aus amorphem Siliciumdioxid im Nano-Maßstab zu modifizieren oder zu synthetisieren. Die gebildeten Strukturen können in der Nanotechnologie angewandt werden.
5.) Verwendung zur Oberflächenmodifikation von Silicium-haltigen Edelsteinen und Halbedelsteinen. Zu den amorphen bzw. feinkristallinen Modifikationen des SiO₂ zählen u. a. Achat, Jaspis und Onyx. Aufgrund der Möglichkeit, mit Hilfe der Silicase unter kontrollierten Bedingungen die Oberfläche dieser Minerale zu modifizieren, ergibt sich die Verwendung des erfindungsgemäßen Verfahrens bei der Herstellung oder Bearbeitung dieser Edelsteine/Halbedelsteine. Hierbei ergibt sich auch die Möglichkeit, gezielt Fremdmoleküle/atome einzufügen.
6. Verwendung bei der Modifikation oder Synthese von Silicium-organischen Verbindungen einschließlich Sila-Pharmaka. Für einen Überblick über die Herstellung von Silicium-organischen Verbindungen als Grundlage für sogenannte Sila-Pharmaka (Pharmaka, bei denen C durch Si ersetzt ist), siehe: Chem. unserer Zeit 14, 197-207 (1980), sowie: Bioactive Organo-Silicon Compounds (Topics Curr. Chem. 84), Berlin, Springer 1979). Mittels des erfindungsgemäßen Verfahrens sind neue, enzymatische Wege zur Modifikation oder Synthese derartiger Verbindungen möglich.

### 8. Verwendungen der Silicase und Silicase-Fusionsproteine zur Prävention und Therapie der Silikose (Quarzstaublungenerkrankung)

### 8.1. Silikose

Kristalline Kieselsäure (Siliciumdioxid) in Form von Quarz, Tridymit oder Cristobalit ist wahrscheinlich einer der wichtigsten Arbeitsplatzgefahrenstoffe. Die Schwere der Gesundheitsbeeinträchtigungen und die Vielfalt der möglichen Expositionsquellen sind lange bekannt. Auf Grund des weitverbreiteten Auftreten von kristallinem Siliciumdioxid in der Erdkruste und des häufigen Gebrauchs von Materialien, die es enthaltenden, sind insbesondere Arbeiter in einer Vielzahl unterschiedlicher Industriebetriebe gegenüber kristallinem Siliciumdioxid ausgesetzt. Es kann davon ausgegangen werden, daß in der Landwirtschaft, im Bergbau, in der Glas- und Faserglasindustrie, bei der Zement-Produktion, bei der Herstellung von Keramiken, in Gießereien, in der Farben-, Seifen- und Kosmetika-Produktion oder in der Dental-Manufaktur/Reparatur Millionen von Beschäftigten regelmäßig gegenüber kristallinem Siliciumdioxid exponiert sind. Nach der "American Thoracic Society" ist Siliciumdioxid weltweit eine der Hauptursachen von Lungenerkrankungen. Somit besteht ein großer Bedarf zur Entwicklung von Strategien zur Prävention und Therapie.

Es ist bekannt, daß inhaliertes kristallines Siliciumdioxid Lungenfibrose (Silikose) und Lungenkrebs verursacht. Die Silikose ist eine maligne Pneumokoniose, die durch eine Akkumulation von Siliciumdioxid-Partikeln im Lungengewebe verursacht wird und durch das Auftreten von silikotischen Knötchen charakterisiert ist. Eine rationale Therapie dieser zu schweren Behinderungen führenden Krankheit existiert nicht.

Ein Hauptgrund für die Toxizität von staubförmigen, kristallinem Siliciumdioxid liegt darin, daß die Lunge nicht in der Lage ist, die eingeatmeten Staubpartikel zu eliminieren. Die im Lungengewebe verbleibenden Siliciumdioxid-Partikel führen zu Entzündungsreaktionen und zur Bildung von zelltoxischen Zytokinen, Proteasen und reaktive Sauerstoffradikalen. Bei Fortdauer dieser Erscheinungen kommt es zu einer Bindegewebsvermehrung mit einer gesteigerten Bildung von Kollagen in der Lunge, was zur Entstehung der Pneumokoniose führt.

Die Silikose entwickelt sich im allgemeinen sehr langsam über Jahrzehnte. Sie ist eine fortschreitende Erkrankung, die nicht heilbar ist. Sie zeigt sich zunächst durch Atemnot, Reizhusten und Stechen in der Brust. Durch Überlastung des Herzens und Einschränkung von Atmung und Kreislauf kommt es schließlich zum Tod. Der durchschnittliche Zeitraum zwischen der Staubexposition und dem Auftreten der Silikose liegt bei etwa 20 Jahre. Eine gefährliche Komplikation der Silikose ist die Silikotuberkulose. Der Mechanismus, der zur Entstehung von Lungenkrebs durch kristallines Siliciumdioxid führt, wird noch wenig verstanden.

Die Silikose ist die häufigste Staublungenerkrankung unter den Berufskrankheiten. Die mittleren Gesamtkosten eines Silikose-Patienten liegen bei etwa 130.000 Euro.

### 8.2. Therapeutika/Protektiva bei Silikose

Die Silicase, die an die an Auflösung von biogenem Siliciumdioxid beteiligt ist, kann als Therapeutikum/Protektivum zur Behandlung der Silikose eingesetzt werden.

Die Silicase ist nicht nur zur Auflösung von amorphem, sondern auch von kristalinem Siliciumdioxid (Quarzkristallen) in der Lage.

Die Silicase besitzt somit die notwendigen Eigenschaften, um Siliciumdioxid aus der Lunge zu eliminieren und/oder den Verlauf dieser Lungenerkrankung zu modulieren.

Verschieden Methoden zur Administration des rekombinanten Enzyms kommen in Betracht: a) als Enzympräparation, b) verpackt in Liposomen, c) assoziiert mit Mikrosphären oder d) Adenovirus-vermittelter Gentransfer.

Mikrosphären als Carrier-Systems für die rekombinante Silicase zur Behandlung der Silikose (Auflösung von SiO₂) können z. B. aus Schwamm-Collagen analog zu Kälber-Callagen-Mikrosphären hergestellt werden (Rössler et al., Pharmazie 49 (1994) 175-179). Die Schwammm-Collagen-Mikropartikel werden durch Adsorption des rekombinanten Proteins (Silicase) beladen, wie beschrieben (Rössler et al., J. Microencapsulation 12 (1995) 49-57; Berthold et al., Eur. J. Pharm. Biopharm. 45 (1998) 23-29). Die Vorteile des Collagens sind seine Bioabbaubarkeit sowie seine niedrige Toxizität und Immunogenität. Als weitere "Delivery"-Systeme kommen u. a. auch Liposomen mit dem eingeschlossenen rekombinanten Enzym sowie Lipid-Nanopartikel in Frage (Jenning et al., Eur. J. Pharm. Biopharm. 49 (2000) 211-218).

### 8.3. Veränderung der Eigenschaften von Zellen durch Transfektion mit einem Silicase-Gen1cDNA-enthaltenden Plasmid

### 8.3. Veränderung der Eigenschaften von Zellen durch Transfektion mit einem Silicase-Gen/cDNA-enthaltenden Plasmid

Durch Transfektion von Zellen mit einem Silicase-Gen/cDNA-enthaltenden Plasmid lassen sich deren Eigenschaften verändern, was u. a. eine Verwendung bei der Herstellung von Knochenersatzmaterialien oder eine Gentherapie (z. B. bei Silikose) ermöglicht.

### Erläuterungen zu den Abbildungen:

Im nachfolgenden sind die erläuternden Legenden zu den beigefügten Abbildungen und dem Sequenzprotokoll aufgeführt. Es zeigen:
SEQ ID No. 1: Die Nukleinsäuresequenz der cDNA der erfindungsgemäßen Silicase aus S. *domuncula.*
SEQ ID No. 2: Die Aminosäuresequenz der erfindungsgemäßen Silicase aus S. *domuncula* (SIA_SUBDO),

### Abbildung 1:

Identifizierung von Transkripten in Primmorphen, die nach Inkubation in 60 µM Silicium für 1 oder 3 Tage hochreguliert wurden, mit Hilfe der. Technik-des Differential Display. Die Primmorphe blieben entweder bei der normalen Silicium-Konzentration von 5 µM (Spur a) oder wurden in Gegenwart von 60 µM Silicium inkubiert (Spur b und c). Die RNA wurde extrahiert und für die Analyse benutzt. Zur Amplifizierung der Transkripte wurden zwei unterschiedliche willkürliche Primer (1 und 2) benutzt. Diejenigen Transkripte, die lediglich bei höherer Silicium-Konzentration (Spur b und c) auftraten und analysiert wurden, sind markiert (>).

### Abbildung 2:

Oben: Aus der Nucleotidsequenz des offenen Leserahmens (codierende Region) der *S. domuncula* Silicase-cDNA (SIA_SUBDO) abgeleitete Aminosäuresequenz. Unten: Nucleotidsequenz der S. *domuncula* Silicase-cDNA (SIA_SUBDO). Die aus der Nucleotidsequenz des offenen Leserahmens abgeleitete Aminosäuresequenz ist unterhalb der Nucleotidsequenz angegeben.
(A) Alignment der S. *domuncula* Silicase (SIA-SUBDO) mit der humanen Carboanhydrase II (Carbonat-Dehydratase II) (CAH2_HUMAN; P00918). Die Carboanhydrase-Domäne ist eingerahmt ( 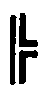 e-CAdorn 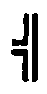 ). Die charakteristischen Aminosäuren, welche die Eukaryonten-Typ-Carboanhydrase-Signatur bilden, sind markiert (▲: in beiden Sequenzen gefunden; ^{a} : lediglich in der Carboanhydrase, aber nicht in der Silicase vorhanden). Die zusätzlichen-Zeichen (+) zeigen diejenigen Reste, die das Hydrogen-Netzwerk des aktiven Zentrums bilden. Die drei Zink-bindenden HistidinReste sind markiert (Z). Ähnliche Aminosäurereste zwischen den beiden Sequenzen sind hervorgehoben. Die Grenzen der langen (~rec~ bis ~rec~) sowie der kurzen rekombinanten Silicase (~rec-s~ bis ~rec~) sind markiert und doppelt unterstrichen. **(B)** Phylogenetischer Baum, konstruiert mit der Schwamm-Silicase und folgenden verwandten Enzymen: humane Carbonanhydrase I (Carbonatdehydratase I) (CAH1_HUMAN; P00915), II (CAH2_HUMAN), III (CAH3_HUMAN; P07451), IV (CAH4_HUMAN; P22748), VI (CAH6_HUMAN; P23280), VII (CAH7_HUMAN; P43166), VIII (CAH8_HUMAN; P35219), IX (CAH9_HUMAN; Q16790), X (CAHA_HUMAN; Q9NS85), VA (CAH5_HUMAN; P35218), VB (CA5B_HUMAN; Q9Y2D0), XII (CAHC_HUMAN; 043570), XIV (CAHE_HUMAN; Q9ULX7), Carboanhydrase von *Caenorhabditis elegans* (CAH_CAEEL; NP_510674.1), Carboanhydrase von *Drosophila melanogaster* (CAH1_DROME; NP_523561.1), Carboanhydrase der Pflanzen *Arabidopsis thaliana* (CAH-I_ARATH; NP_196038.1) und *Chlamydomonas reinhardtii* (Carbonatdehydratase 1) (CAH1_CHLRE; P20507) sowie bakterielle Carboanhydrasen von *Neisseria gonorrhoeae* (CAH_NEIGO; Q50940), *Klebsiella pneumoniae* (CAH_KLEPN; 052535) und den Cyanobakterien *Nostoc sp.* PCC 7120 (CAH_ANASP; P94170). Die letztgenannte Sequenz diente als Outgroup. Die Meßbalken zeigen eine evolutionäre Distanz von 0,1 Aminosäure-Substitutionen pro Position in der Sequenz an. Der phylogenetische Baum wurde mittels "Neighbour-Joining" konstruiert ("Neighbor" Programm: Felsenstein, J. (1993). PHYLIP, ver. 3.5. University of Washington, Seattle).

### Abbildung 4:

Herstellung der rekombinanten Silicase. Die rekombinante S. *domuncula* Silicase (rSIA-SUBDO) wurde als GST-Fusionsprotein hergestellt. Sowohl die lange als auch die kurze *SDSIA* wurden in ein pGEX-4T-2-Plasmid kloniert, das das Glutathion S-Transferase (GST)-Gen enthielt. Die Fusionsproteine wurden entweder ohne vorhergehende Induktion mit IPTG (-IPTG) oder nach Inkubation mit IPTG (+IPTG) für 4 oder 6 Stunden isoliert, danach gespalten, gereinigt und anschließend der Na-DodSO₄-PAGE unterworfen. Das Gel wurde mit Coomassie Brillant Blau gefärbt. Die gereinigte lange Form rSIA-SUBDO-1 mit einer Größe von 43 kDa sowie die kurze Form (Mᵣ 32 kDa) der Silicase wurden erhalten.

### Abbildung 5:

Bestimmung der Enzymaktivität der Silicase im Carboanhydrase- und im Silicase-Assay. Die rekombinante Silicase wurde zu den Reaktionsmischungen hinzugefügt in Konzentrationen zwischen 1 und 10 µg pro Assay (0,56 µl). Zur Bestimmung der Carboanhydrase-Aktivität (■) wurde *p*-Nitrophenylacetat als Substrat benutzt. Das freigesetzte *p*-Nitrophenol wurde bei einer Wellenlänge von 348 nm gemessen. Die Aktivität der Silicase (●) wurde unter Verwendung von *S. domuncula* Spiculae bestimmt. Die freigesetzte, durch Depolymerisation (Abbau) von amorphen Siliciumdioxid gebildete Kieselsäure wurde mit Hilfe der "Silicon Test" kolorimetrischen Reaktion bestimmt.

### Abbildung 6:

Effekt von Silicium auf die Bildung von Spiculae in Primmorphen. Für die Bildung der Primmorphe wurden dissoziierte Zellen des Meeresschwammes S. *domuncula* in Seewasser, ergänzt mit 10% RPMI1640-Medium und 30 µM Fe(+++), inkubiert. Die Primmorphe wurden dann für 3 Tage in ein Medium (RPMI 1640, Fe(+++)), das mit 60 µM Silicium angereichert wurde, überführt. (A) Die Primmorphe wurden in Medium plus Silicium inkubiert. Vergrößerung: x6. (B) In einigen Fällen begannen die Primmorphe mit der Synthese von Spiculae (sp). Vergrößerung: x10. Zur semiquantitativen Messung wurden die Primmorphe zwischen zwei Deckgläser gepresst (C und D). (C) Primmorphe, die in Abwesenheit von Silicium inkubiert wurden, waren fast komplett ohne Spiculae, während diejenigen, die in Gegenwart von Silicium kultiviert wurden, neugebildete Spiculae enthielten (>) (D); Vergrößerung: x200.

### Abbildung 7:

Expression der Silicase, des Silicateins und der Isocitrat-Dehydrogenase, bestimmt durch Northern-Blotting. Die RNA wurde aus Primmorphen extrahiert, die in Abwesenheit von zusätzlichem Silicium (- Si) oder in Gegenwart von 60 µM Silicium (+ Si) für 1 bis 3 Tage inkubiert worden waren. 5 µg der Gesamt-RNA wurde elektrophoretisch aufgetrennt, auf Nylonmembranen geblottet und mit den folgenden Sonden hybridisiert: *SDSIA* (Silicase), *SDSILICA* (Silicatein) und *SDIDH* (α-Untereinheit der Isocitrat-Dehydrogenase). Die Größen der Transkripte sind angegeben.

### Abbildung 8:

Enzymatische, von der Silicase (Carboanhydrase) von S. *domuncula* vermittelte Reaktionen. In [1] ist die Überführung von CO₂ in HCO³⁻ gezeigt. In [2] ist die Reaktion der Silicase gezeigt. Die Silicase bindet mit ihren drei Histidin-Resten ein Zinkatom. Das Zinkion, eine Lewis-Säure, bindet ein Hydroxid-Anion, das aus Wasser, einer Lewis-Base, stammt. Der Silicase/Zink-Komplex unternimmt einen nucleophilen Angriff auf ein Siliciumatom zwischen den Sauerstoffbindungen. Dadurch wird die Hydrolyse des polymeren Siliciumdoxids bewirkt, das zunächst - mit einer der beiden Produkthälften - an das Enzym gebunden bleibt. Unter weiterem Verbauch von H₂O wird das Produkt freigesetzt bis nach mehreren Cyclen letztendlich freie Kieselsäure zurückbleibt.

### Abbildung 9:

Links: Spiculae (Nadeln) von *Suberites domuncula* nach 6 stündiger Inkubation in Abwesenheit von Silicase. Rechts: Spiculae von *Suberites domuncula* nach 6 stündiger Inkubation in Gegenwart von Silicase. Die Inkubation erfolgte unter den in Tabelle 2 beschriebenen Bedingungen.

## Patentansprüche

1. Verfahren zum *in vitro* Abbau von amorphem oder kristallinem Siliciumdioxid, Siliconen und anderen Silicium(IV)- oder Metall (IV)-Verbindungen sowie von Mischpolymeren dieser Verbindungen, wobei ein Polypeptid oder ein Metallkomplex eines Polypeptids zum Abbau eingesetzt wird,
**dadurch gekennzeichnet, daß**
a) das Polypeptid eine tierische, bakterielle, pflanzliche oder Pilz Carboanhydrase-Domäne umfaßt, die mindestens 25 % Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.

2. Verfahren zur Synthese von amorphem Siliciumdioxid, Siliconen und anderen Silicium (IV)- oder Metall (IV)-Verbindungen sowie von Mischpolymeren dieser Verbindungen, wobei ein Polypeptid oder ein Metallkomplex eines Polypeptids zur Synthese eingesetzt wird, **dadurch gekennzeichnet, daß**
a) das Polypeptid eine tierische, bakterielle, pflanzliche oder Pilz Carboanhydrase-Domäne umfaßt, die mindestens 25 % Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.

3. Verfahren nach Anspruch 2, **dadurch gekennzeichnet, daß** zur Synthese Verbindungen wie Kieselsäuren, Monoalkoxysilantriole, Dialkoxysilandiole, Trialkoxysilanole, Tetraalkoxysilane, Alkyl- oder Aryl-Silantriole, Alkyl- oder Aryl-Monoalkoxysilandiole, Alkyl- oder Aryl-Dialkoxysilanole, Alkyl- oder Aryl-Trialkoxysilane oder andere Metall(IV)-Verbindungen als Reaktanten eingesetzt werden.

4. Verfahren nach Anspruch 3, wobei durch Verwendung definierter Mischungen der Verbindungen Mischpolymere definierter Zusammensetzung hergestellt werden.

5. Verfahren nach einem der Ansprüche 2 bis 4, wobei durch das Polypeptid oder einen Metallkomplex des Polypeptids oder die Bindung des Polypeptids oder eines Metallkomplexes des Polypeptids an andere Moleküle oder die Oberflächen von Glas, Metallen, Metalloxiden, Kunststoffen, Biopolymeren oder anderen Materialien als Template die Ausbildung definierter zwei- und dreidimensionaler Strukturen erfolgt.

6. Verfahren zur Modifikation einer Kieselsäure oder Silicium(IV)- oder Metall(IV)-Verbindung enthaltenden Struktur oder Oberfläche, wobei ein Polypeptid oder ein Metallkomplex eines Polypeptids zur Modifikation eingesetzt wird, **dadurch gekennzeichnet, daß**
a) das Polypeptid eine tierische, bakterielle, pflanzliche oder Pilz Carboanhydrase-Domäne umfaßt, die mindestens 25 % Sequenzähnlichkeit zu der in SEQ ID Nr. 2 gezeigten Sequenz aufweist,
b) das Polypeptid Carboanhydrase-Aktivität aufweist und
c) das Polypeptid in der Lage ist, sowohl amorphes als auch kristallines Siliciumdioxid aufzulösen.

7. Verfahren nach Anspruch 6, wobei die Kieselsäure enthaltende Struktur oder Oberfläche in Form eines Edelsteins oder Halbedelsteins vorliegt.

8. Verfahren nach Anspruch 6 oder 7, wobei die Modifikation eine Glättung, ein Anätzen oder ein Herstellen von Ausbohrungen der Kieselsäure oder Silicium(IV)- oder Metall(IV)-Verbindung enthaltenden Struktur oder Oberfläche durch das Polypeptid oder einen Metallkomplex des Polypeptids umfaßt.

9. Polypeptid einer Silicase aus *Suberites domuncula* gemäß SEQ ID Nr. 2 oder ein dazu homologes Polypeptid, das
a) eine tierische, bakterielle, pflanzliche oder Pilz Carboanhydrase-Domäne umfaßt, die mindestens 25 % Sequenzähnlichkeit zu der in SEQ ID Nr. 2 gezeigten Sequenz aufweist,
b) Carboanhydrase-Aktivität aufweist und
c) in der Lage ist, sowohl amorphes als auch kristallines Siliciumdioxid aufzulösen, oder ein Metallkomplex dieses Polypeptids.

10. Nukleinsäure, insbesondere gemäß SEQ ID Nr. 1, **dadurch gekennzeichnet, daß** sie für ein Polypeptid gemäß Anspruch 9 kodiert.

11. Nukleinsäure nach Anspruch 10 in Form eines (a) Fusionsprotein-Konstrutkts, (b) Konstrukts mit getrennter Protein-Expression oder (c) Konstrukts mit getrennter Protein-Expression.

12. Nukleinsäure nach einem der Ansprüche 10 oder 11, **dadurch gekennzeichnet, daß** die Nukleinsäure synthetisch hergestellt worden ist.

13. Vektor, umfassend eine Nukleinsäure nach einem der Ansprüche 10 bis 12.

14. Vektor nach Anspruch 13, ausgewählt aus einem Plasmid, shuttle Vektor, Phagemid, Cosmid, Expressionsvektor, retroviralen Vektor, adenoviralen Vektor.

15. Verwendung eines Polypeptids oder einer Nukleinsäure nach einem der voranstehenden Ansprüche zur Herstellung eines Medikaments zur Prävention oder Therapie der Silikose oder zur Modulation der Resorbierbarkeit von Siliconen und Silicon-Implantaten.

## Claims

1. Method for the *in vitro* degradation of amorphous or crystalline silicon dioxide, silicones and other silicon(IV)- or metal(IV)-compounds as well as mix-polymers of these compounds, wherein a polypeptide or a metal complex of the polypeptide is used for said degradation,
**characterized in that**
a) the polypeptide comprises an animal, bacterial, plant or fungal carboanhydrase that has at least 25 % sequence similarity to the sequence as shown in SEQ ID No. 2,
b) the polypeptide has carboanhydrase-activity, and
c) the polypeptide is able to dissolve both amorphous as well as crystalline silicon dioxide.

2. Method for the synthesis of amorphous or crystalline silicon dioxide, silicones and other silicon(IV)- or metal(IV)-compounds as well as mix-polymers of these compounds, wherein a polypeptide or a metal complex of the polypeptide is used for said synthesis,
**characterized in that**
a) the polypeptide comprises an animal, bacterial, plant or fungal carboanhydrase that has at least 25 % sequence similarity to the sequence as shown in SEQ ID No. 2,
b) the polypeptide has carboanhydrase-activity, and
c) the polypeptide is able to dissolve both amorphous as well as crystalline silicon dioxide.

3. Method according to claim 2, **characterized in that** compounds such as silicon dioxides, monoalkoxy silantrioles, dialkoxy silandioles, trialkoxy silanoles, tetraalkoxy silanes, alkyl- or aryl-silantrioles, alkyl- or aryl-monoalkoxy silandioles, alkyl- or aryl-dialkoxy silanoles, alkyl- or aryl-trialkoxy silanes or other metal(IV)-compounds are used as reactants for the synthesis.

4. Method according to claim 3, wherein defined compositions are produced by using defined mixtures of the compounds.

5. Method according to any of claims 2 to 4, wherein the formation of defined two- and three-dimensional structures occurs through the polypeptide or a metal complex of said polypeptide or the binding of said polypeptide or a metal complex of said polypeptide to other molecules or the surfaces of glass, metals, metal oxides, plastics, biopolymers or other materials as templates.

6. Method for the modification of a silicon dioxide or silicon(IV)- or metal(IV)-compound containing structure or surface, wherein a polypeptide or a metal complex of the polypeptide is used for said modification,
**characterized in that**
a) the polypeptide comprises an animal, bacterial, plant or fungal carboanhydrase that has at least 25 % sequence similarity to the sequence as shown in SEQ ID No. 2,
b) the polypeptide has carboanhydrase-activity, and
c) the polypeptide is able to dissolve both amorphous as well as crystalline silicon dioxide.

7. Method according to claim 6, wherein the silicon dioxide containing structure or surface is present in form of a precious stone or a semi-precious stone.

8. Method according to claim 6 or 7, wherein the modification comprises a smoothening, a slight etching or the production of boreholes of the silicon dioxide or silicon(IV)- or metal(IV)-compound containing structure or surface by the polypeptide or a metal complex of said polypeptide.

9. Polypeptide of a silicase from *Suberites domuncula* according to SEQ ID No. 2 or a polypeptide being homologous thereto, which
a) comprises an animal, bacterial, plant or fungal carboanhydrase that has at least 25 % sequence similarity to the sequence as shown in SEQ ID No. 2,
b) has carboanhydrase-activity, and
c) is able to dissolve both amorphous as well as crystalline silicon dioxide, or a metal complex of said polypeptide.

10. Nucleic acid, in particular according to SEQ ID No. 1, **characterized in that** it encodes for a polypeptide according to claim 9.

11. Nucleic acid according to claim 10 in form of a (a) fusion-protein-construct, (b) construct with separate protein-expression or (c) construct with separate protein-expression.

12. Nucleic acid according to any one of claims 10 or 11, **characterized in that** the nucleic acid was produced synthetically.

13. Vector, comprising a nucleic acid according to any one of claims 10 to 12.

14. Vector according to claim 13, selected from a plasmid, shuttle vector, phagemid, cosmid, expression vector, retroviral vector, adenoviral vector.

15. Use of a polypeptide or a nucleic acid according to any of the preceding claims for the production of a medicament for the prevention or therapy of silicosis or for the modulation of the resorbability of silicones and silicone-implants.

## Revendications

1. Méthode de dégradation *in vitro* de dioxyde de silicium amorphe ou cristallin, de silicones et d'autres composés de silicium (IV) ou de métal (IV) ainsi que des mélanges polymères de ces composés, dans laquelle un polypeptide ou un complexe métallique du polypeptide est utilisé pour ladite dégradation,
**caractérisée en ce que**
a) le polypeptide comprend une carboanhydrase animale, bactérienne, végétale ou fongique qui a au moins une similarité de séquence de 25 % par rapport à la séquence illustrée dans SEQ ID n° 2,
b) le polypeptide a une activité de carboanhydrase, et
c) le polypeptide est capable de dissoudre à la fois le dioxyde de silicium amorphe et cristallin.

2. Méthode de synthèse de dioxyde de silicium amorphe ou cristallin, de silicones et d'autres composés de silicium (IV) ou de métal (IV) ainsi que des mélanges polymères de ces composés, dans laquelle un polypeptide ou un complexe métallique du polypeptide est utilisé pour ladite synthèse,
**caractérisée en ce que**
a) le polypeptide comprend une carboanhydrase animale, bactérienne, végétale ou fongique qui a au moins une similarité de séquence de 25 % par rapport à la séquence illustrée dans SEQ ID n° 2,
b) le polypeptide a une activité de carboanhydrase, et
c) le polypeptide est capable de dissoudre à la fois le dioxyde de silicium amorphe et cristallin.

3. Méthode selon la revendication 2, **caractérisée en ce que** les composés tels que les dioxydes de silicium, les monoalcoxy silantrioles, les dialcoxy silandioles, les trialcoxy silanoles, les tétraalcoxy silanes, les alkyl- ou aryl-silantrioles, les alkyl- ou aryl-monoalcoxy silandioles, les alkyl- ou aryl-dialcoxy silanoles, les alkyl- ou aryl-trialcoxy silanes ou d'autres composés de métal (IV) sont utilisés en tant que réactifs pour la synthèse.

4. Méthode selon la revendication 3, dans laquelle les compositions définies sont produites en utilisant des mélanges définis des composés.

5. Méthode selon l'une quelconque des revendications 2 à 4, dans laquelle la formation des structures bi- et tri-dimensionnelles définies se produit par le polypeptide ou un complexe métallique dudit polypeptide ou la liaison dudit polypeptide ou d'un complexe métallique dudit polypeptide à d'autres molécules ou aux surfaces du verre, de métaux, d'oxydes métalliques, de plastiques, de biopolymères ou d'autres matériaux en tant que matrices.

6. Méthode de modification d'un dioxyde de silicium ou d'une structure ou d'une surface contenant un composé de silicium (IV) ou de métal (IV), dans laquelle un polypeptide ou un complexe métallique du polypeptide est utilisé pour ladite modification,
**caractérisée en ce que**
a) le polypeptide comprend une carboanhydrase animale, bactérienne, végétale ou fongique qui a au moins une similarité de séquence de 25 % par rapport à la séquence illustrée dans SEQ ID n° 2,
b) le polypeptide a une activité de carboanhydrase, et
c) le polypeptide est capable de dissoudre à la fois le dioxyde de silicium amorphe et cristallin.

7. Méthode selon la revendication 6, dans laquelle la structure ou surface contenant du dioxyde de silicium est présente sous la forme d'une pierre précieuse ou d'une pierre semi-précieuse.

8. Méthode selon la revendication 6 ou 7, dans laquelle la modification comprend un lissage, un léger décapage ou la production de pores de dioxyde de silicium ou d'une structure ou surface contenant un composé de silicium (IV) ou de métal (IV) par le polypeptide ou un complexe métallique dudit polypeptide.

9. Polypeptide d'une silicase de *Suberites domuncula* selon la SEQ ID n° 2 ou un polypeptide homologue à celui-ci, qui
a) comprend une carboanhydrase animale, bactérienne, végétale ou fongique qui a au moins une similarité de séquence de 25 % par rapport à la séquence illustrée dans SEQ ID n° 2,
b) a une activité de carboanhydrase, et
c) est capable de dissoudre à la fois le dioxyde de silicium amorphe et cristallin,
ou un complexe métallique dudit polypeptide.

10. Acide nucléique, en particulier selon la SEQ ID n° 1, **caractérisé en ce qu'**il code un polypeptide selon la revendication 9.

11. Acide nucléique selon la revendication 10 sous la forme d'un (a) concept de type protéine hybride, (b) concept avec une expression protéique séparée ou (c) un concept avec une expression protéique séparée.

12. Acide nucléique selon l'une quelconque des revendications 10 et 11, **caractérisé en ce que** l'acide nucléique est produit de manière synthétique.

13. Vecteur comprenant un acide nucléique selon l'une quelconque des revendications 10 à 12.

14. Vecteur selon la revendication 13, choisi parmi un plasmide, un vecteur navette, un phagemide, un cosmide, un vecteur d'expression, un vecteur rétroviral, un vecteur adénoviral.

15. Utilisation d'un polypeptide ou d'un acide nucléique selon l'une quelconque des revendications précédentes pour la production d'un médicament destiné à la prévention ou le traitement d'une silicose ou à la modulation de la résorbabilité de silicones et d'implants de silicone.
